# EUROPEAN PATENT APPLICATION

(11) **EP 2 000 456 A2**
(43) Date of publication of application: **10.12.2008**
(21) Application number: 07739864.2
(22) Date of filing: 27.03.2007
(51) Int. Cl.: C07C 50/28, C07C 50/32, C07C 255/37, C07C 261/04, C09K 11/06, H01L 51/50

(54) **MATERIAL FOR ORGANIC ELECTROLUMINESCENT DEVICE AND ORGANIC ELECTROLUMINESCENT DEVICE USING THE SAME**

(30) Priority: 30.03.2006 JP 2006094470
(71) Applicant: Idemitsu Kosan Co., Ltd., Chiyoda-ku Tokyo 100-8321 (JP)
(72) Inventor: MORISHITA, Hironobu, Sodegaura-shi, Chiba 299-0293 (JP); KAWAMURA, Hisayuki, Sodegaura-shi, Chiba 299-0293 (JP); HOSOKAWA, Chishio, Sodegaura-shi, Chiba 299-0293 (JP)
(74) Representative: Gille Hrabal Struck Neidlein Prop Roos
(86) International application number: PCT/JP2007/056426
(87) International publication number: WO 2007/116750

(57) **Abstract**

A material for an organic electroluminescent device inclusing a quinone derivative represented by the following formula (1), (2) or (3): 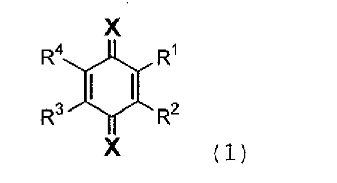 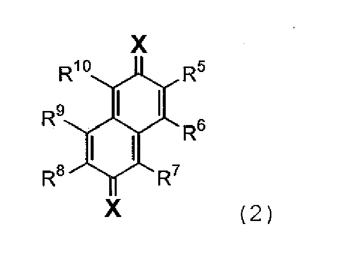 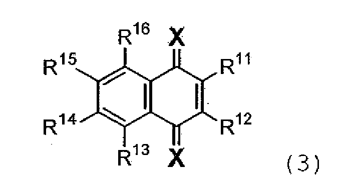 wherein R¹ to R¹⁶ are each a hydrogen atom, a halogen atom, a cyano group, an alkoxy group, a substituted or unsubstituted aryloxy group, an alkyl group, a fluoroalkyl group, an aryl group or a heterocyclic group; provided that at least one of R¹ to R⁴, at least one of R⁵ to R¹⁰ or at least one of R¹¹ to R¹⁶ is an aryloxy group; and X is a substituent represented by any one of the following formulas (a) to (f): 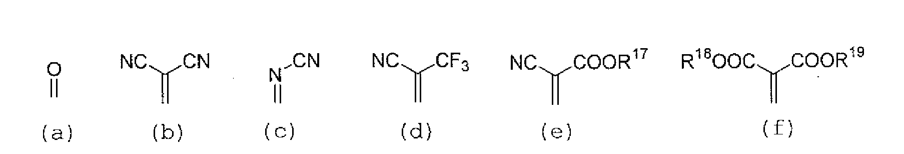 wherein R¹⁷ to R¹⁹ are a hydrogen atom, an alkyl group, or aryl group; and R¹⁸ and R¹⁹ may be bonded together to form a ring.

## Description

### TECHNICAL FIELD

The invention relates to a material for an organic electroluminescent device and an organic electroluminescent device using the same.

### BACKGROUND

An organic electroluminescent (hereinafter "electroluminescent" is often abbreviated as "EL") device is a self-emission device by the use of the principle that a fluorescent compound emits light by the recombination energy of holes injected from an anode and electrons injected from a cathode when an electric field is impressed.

Since C. W. Tang et al. of Eastman Kodak Co. reported a low-voltage driven organic EL device in the form of a stacked type device (Non-patent Document 1, or the like), studies on organic EL devices wherein organic materials are used as the constituent materials has actively been conducted.
The organic EL device reported by Tang et al. has a stacked structure in which tris(8-hydroxyquinolinol)aluminum is used as an emitting layer and a triphenyldiamine derivative is used as a hole-transporting layer. The advantages of the stack structure are to increase injection efficiency of holes to the emitting layer, to increase generation efficiency of excitons generated by recombination by blocking electrons injected in the cathode, to confine the generated excitons in the emitting layer, and so on.

Like this example, as the structure of the organic EL device, a two-layered type of a hole-transporting (injecting) layer and an electron-transporting emitting layer, and a three-layered type of a hole-transporting (injecting) layer, an emitting layer and an electron-transporting (injecting) layer are widely known. In such stack structure devices, their device structures and fabrication methods have been contrived to increase recombination efficiency of injected holes and electrons.

Heretofore, an aromatic diamine derivative as described in Patent Document 1 or an aromatic condensed ring diamine derivative as described in Patent Document 2 is known as a hole-transporting material used in an organic EL device.
In an organic EL device using the aromatic diamine derivative as the hole-transporting material, a high applied voltage is required in order to obtain a sufficient luminance. Applying a high voltage causes such problems as shortened lifetime of the device, increased power consumption, and the like.

To solve the problems, doping a hole-injection layer with an electron-accepting compound such as Lewis acid or the like has been proposed (Patent Documents 3 to 6, or the like). However, the electron-accepting compounds used in those Patent Documents have disadvantages that they are unstable to handle during fabricating an organic EL device, that the lifetime of an organic EL device fabricated using these compounds is shortened due to a lowering in stability such as heat resistance when an organic EL device is driven, and the like.
Tetrafluorodicyanoquinodimethane of an electron-accepting compound described in Patent Documents 5, 7 ,8 and the like is sublimed readily since it has a low molecular weight and is substituted with fluorine. Therefore, tetrafluorodicyanoquinodimethane may diffuse within an apparatus when fabricating an organic EL device by vacuum deposition, causing the apparatus or the device to be contaminated. In addition, it is crystallized when forming a device therefrom to cause current leakage.
[Patent Document 1] United States Patent No. 4,720,432
[Patent Document 2] United States Patent No. 5,061,569
[Patent Document 3] JP-A-2003-031365
[Patent Document 4] JP-A-2001-297883
[Patent Document 5] JP-A-2000-196140
[Patent Document 6] JP-A-11-251067
[Patent Document 7] JP-A-4-297076
[Patent Document 8] JP-T-2004-514257
[Non-patent document 1] C. W. Tang, S. A. Vanslyke, Applied Physics Letters, 51, 913 (1987)

The invention has been made based on the above problems. An object of the invention is to provide an organic EL device which can be driven at a low voltage and have a long lifetime.

### DISCLOSURE OF THE INVENTION

The inventors made extensive studies and have found that, by introducing an aryloxy group such as a phenoxy group into benzoquinone or naphthoquinone of an electron-accepting compound, the electron-accepting compound retains high electron-accepting properties, and has improved heat resistance and crystallizing suppression. The inventors have found that an organic EL device using these quinone derivatives can be driven at a low voltage and can exhibit a long lifetime.

The invention provides the following material for an organic electroluminescent device, and the like.
1. A material for an organic electroluminescent device comprising a quinone derivative represented by the following formula (1), (2) or (3): 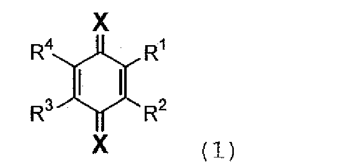 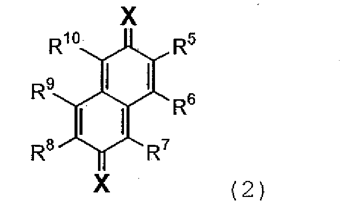 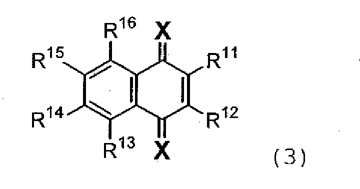 wherein R¹ to R¹⁶ are each a hydrogen atom, a halogen atom, a cyano group, an alkoxy group, a substituted or unsubstituted aryloxy group, an alkyl group, a fluoroalkyl group, an aryl group or a heterocyclic group; provided that at least one of R¹ to R⁴, at least one of R⁵ to R¹⁰ or at least one of R¹¹ to R¹⁶ is an aryloxy group; and X is a substituent represented by any one of the following formulas (a) to (f): 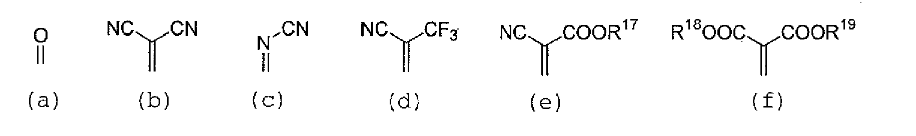 wherein R¹⁷ to R¹⁹ are a hydrogen atom, an alkyl group, or aryl group; and R¹⁸ and R¹⁹ may be bonded together to form a ring.
2. An organic electroluminescent device comprising:
   an anode,
   a cathode, and
   one or a plurality of organic thin layer(s) comprising an emitting layer, interposed between the anode and the cathode;
   at least one layer of the organic thin layer(s) containing the material for an organic electroluminescent device of 1.
3. The organic electroluminescent device according to 2 wherein the organic thin layers are a multilayer body in which a hole-transporting layer, an emitting layer and an electron-transporting layer are stacked in this order from the anode.
4. The organic electroluminescent device according to 3 wherein the hole-transporting layer contains the material for an organic electroluminescent device.
5. The organic electroluminescent device according to 2 wherein the organic thin layers are a multilayer body in which a hole-injecting layer, a hole-transporting layer, an emitting layer and an electron-transporting layer are stacked in this order from the anode;
   the hole-injecting layer containing the material for an organic electroluminescent device.
6. The organic electroluminescent device according to 4 or 5 wherein the hole-transporting layer or the hole-injecting layer containing the material for an organic electroluminescent device further contains a phenylenediamine compound represented by the following formula (4): 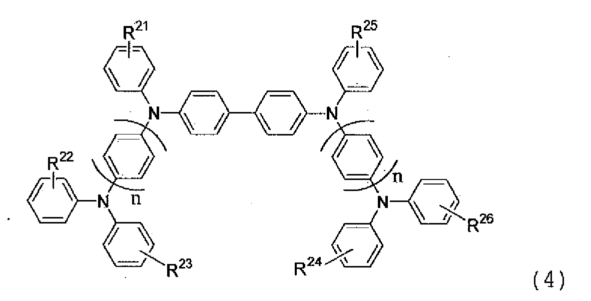 wherein R²¹ to R²⁶ are a hydrogen atom, a halogen atom, a trifluoromethyl group, an alkyl group, an aryl group or a heterocyclic group; R²¹ to R²⁶ may form a naphthalene skeleton, a carbazole skeleton, or a fluorene skeleton with a phenyl group bonded; and n represents 1 or 2.
7. A quinone derivative represented by the following formula (5), (6) or (7): 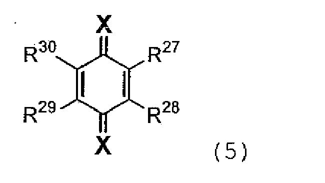 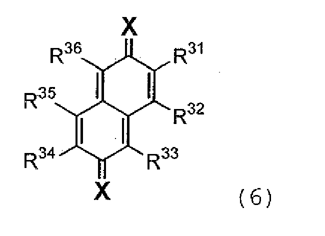 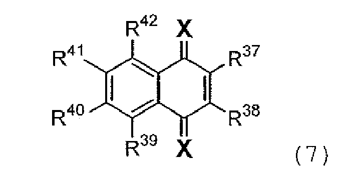
wherein R²⁷ to R⁴² are each a hydrogen atom, a halogen atom, a cyano group, an alkoxy group, a substituted or unsubstituted aryloxy group, an alkyl group, a fluoroalkyl group, an aryl group or a heterocyclic group; provided that at least one of R²⁷ to R³⁰, at least one of R³¹ to R³⁶ or at least one of R³⁷ to R⁴² is a fluorine atom or an aryloxy group having a fluoroalkyl group; and X is a substituent represented by any one of the following formulas (a) to (f) : 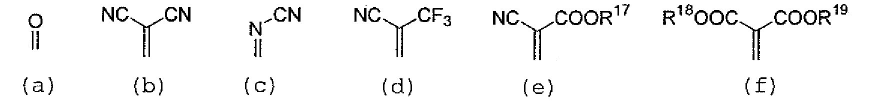 wherein R¹⁷ to R¹⁹ are a hydrogen atom, an alkyl group, or aryl group; and R¹⁸ and R¹⁹ may be bonded together to form a ring.

According to the invention, a material for an organic EL device containing a quinone derivative, which has high electron-accepting properties and lowered crystallinity, is provided. Also, according to the invention, an organic EL device which can be driven at a low voltage and has a long lifetime is provided.

### BRIEF DESCRIPTION OF DRAWINGS

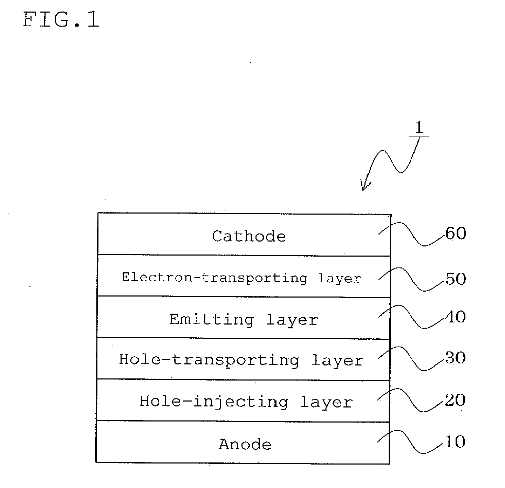

FIG. 1 is a cross-sectional view showing an embodiment of an organic EL device according to the invention.

### BEST MODE FOR CARRYING OUT THE INVENTION

Firstly, the quinone derivative contained in the material for an organic EL device of the invention will be described (hereinafter referred to as "quinone derivative of the invention").
One of the quinone derivatives of the invention is a benzoquinone derivative represented by the following formula (1):

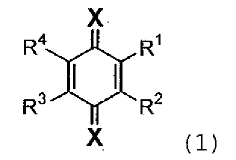

wherein R¹ to R⁴ are each a hydrogen atom, a halogen atom, a cyano group, an alkoxy group, a substituted or unsubstituted aryloxy group, an alkyl group, a fluoroalkyl group, an aryl group or a heterocyclic group; provided that at least one of R¹ to R⁴ is an aryloxy group.

wherein X is a substituent represented by any one of the following formulas (a) to (f); and when X is a substituent represented by any one of the formulas (c) to (f), the benzoquinone derivative represented by the formula (1) has isomers (in cis form and trans form), but the benzoquinone derivative may exist in cis form, trans form or a mixture thereof: 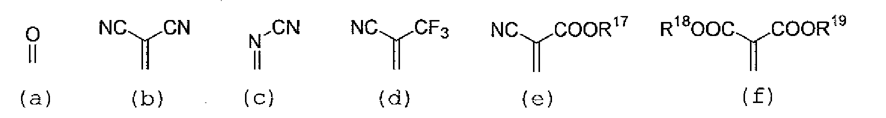 wherein R¹⁷ to R¹⁹ are a hydrogen atom, an alkyl group, or aryl group; and R¹⁸ and R¹⁹ may be bonded together to form a ring.

As the halogen for R¹ to R⁴, fluorine and chlorine are preferable.

As the alkoxy group for R¹ to R⁴, a methoxy group and ethoxy group are preferable.

As the aryloxy group for R¹ to R⁴, a substituted or unsubstituted phenoxy group is preferable. As the substituent of the phenoxy group, an electron-withdrawing group such as a halogen atom including fluorine, a trifluoromethyl group and a cyano group is preferable.

As the alkyl group for R¹ to R⁴, a methyl group and an ethyl group are preferable.

As the fluoroalkyl group for R¹ to R⁴, a trifluoromethyl group and a pentafluoroethyl group are preferable.

As the aryl group for R¹ to R⁴, a phenyl group and a tolyl group are preferable.

As the heterocyclic group for R¹ to R⁴, pyridine, pyrazine and benzofuran are preferable.

As the alkyl group for R¹⁷ to R¹⁹, a methyl group, an ethyl group, a propyl group and tert-butyl group are preferable.

As the aryl group for R¹⁷ to R¹⁹, a phenyl group and a tolyl group are preferable.

R¹⁷ and R¹⁸ may also be bonded together to form a ring.
For example, the following ring structure can be formed. 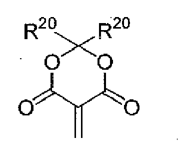 wherein R²⁰s are each a methyl group, a propyl group or a tert-butyl group.

Preferable examples of the benzoquinone derivative represented by the formula (1) are shown below:

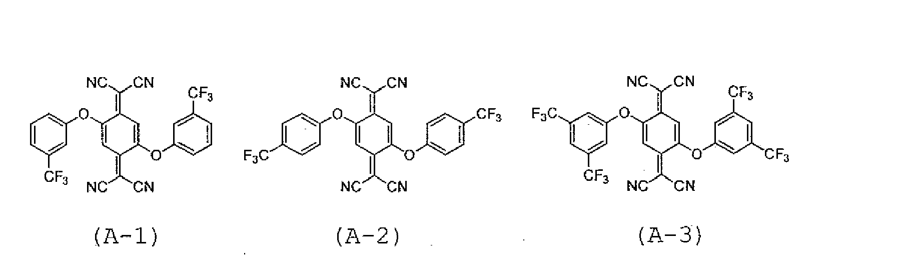

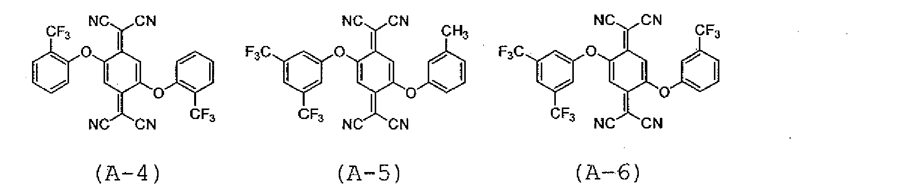

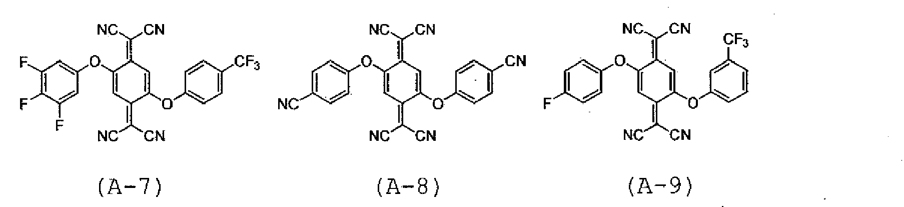

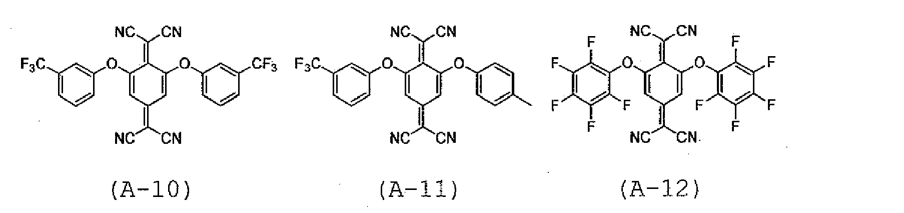

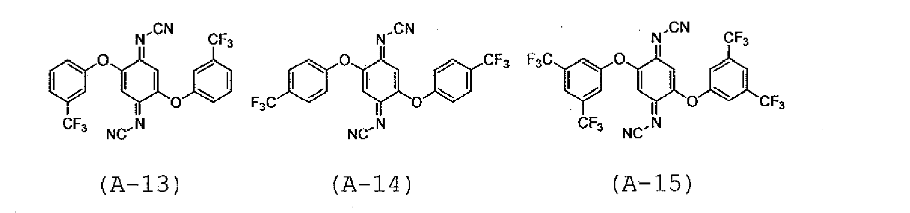

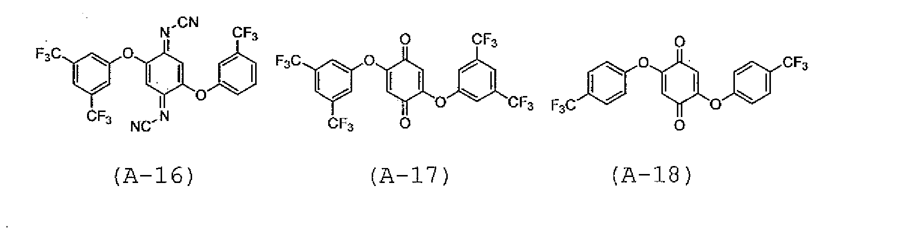

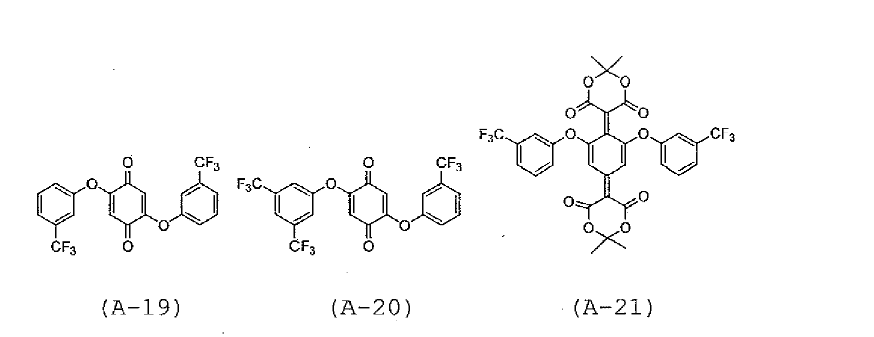

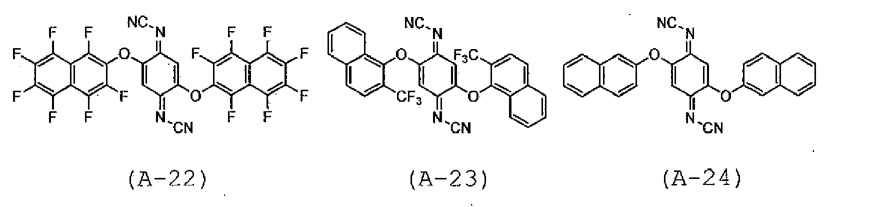

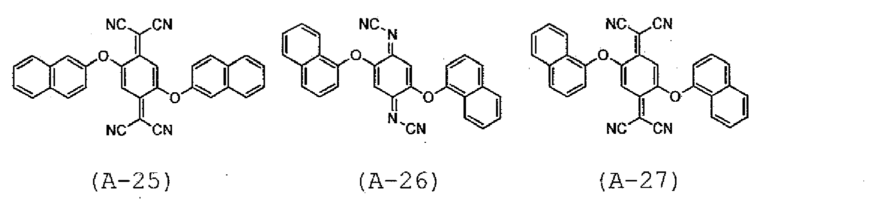

The quinone derivative of the invention is a naphthoquinone derivative represented by the following formula (2):

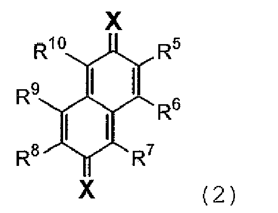

wherein R⁵ to R¹⁰ are the same as R¹ to R⁴ of the formula (2); and X is the same as X of the formula (1).

Preferable examples of the naphthoquinone derivative represented by the formula (2) are shown below:

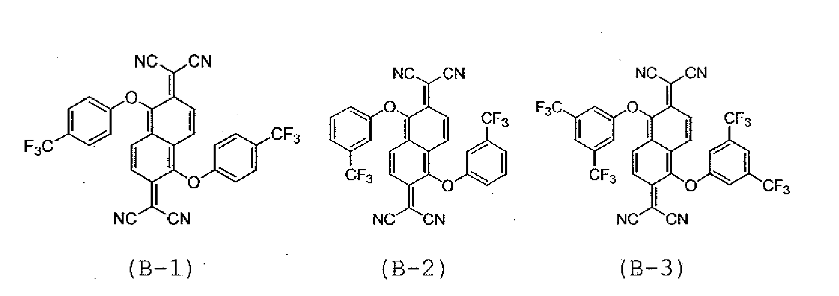

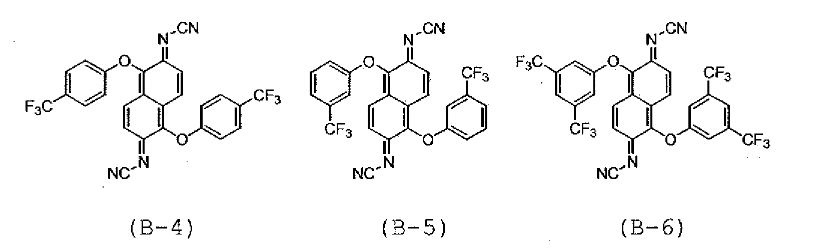

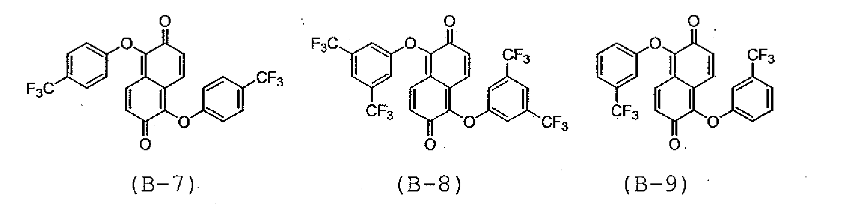

The quinone derivative of the invention is a naphthoquinone derivative represented by the following formula (3):

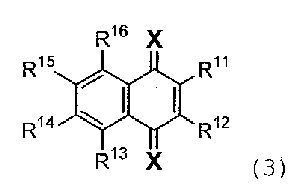

wherein R¹¹ to R¹⁶ are the same as R¹ to R⁴ of the formula (3); and X is the same as X of the formula (1).

Preferable examples of the naphthoquinone derivative represented by the formula (3) are shown below:

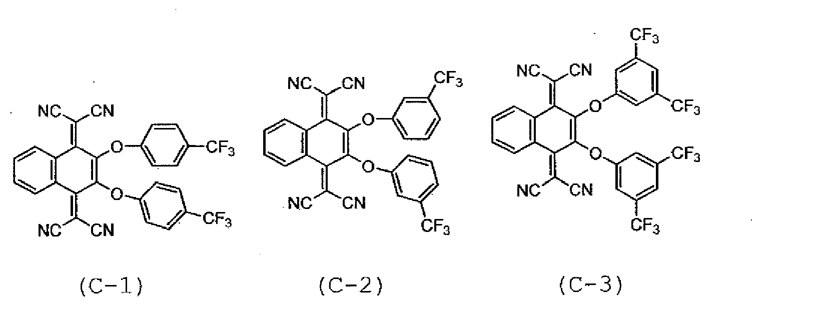

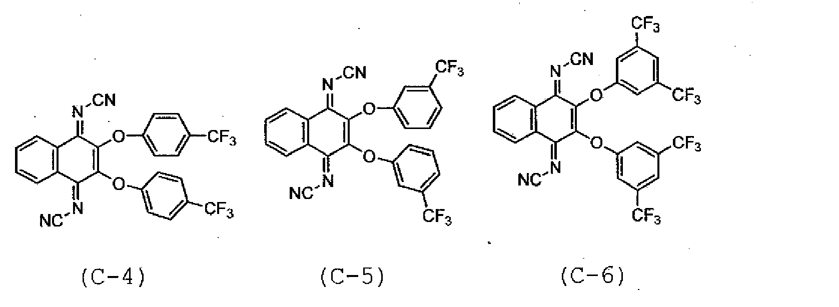

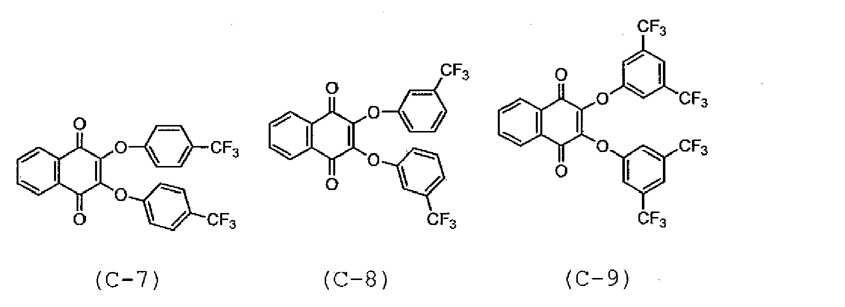

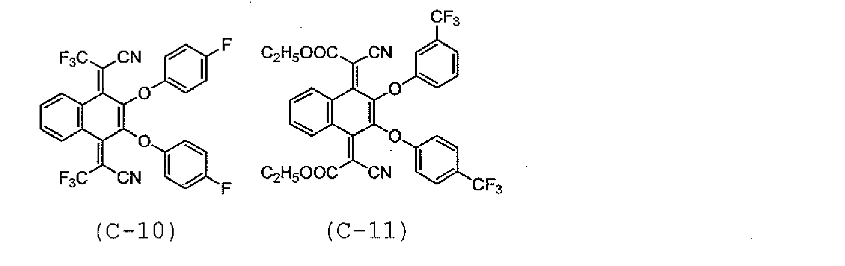

As a novel quinone derivative, the quinone derivatives represented by the following formulas (5) to (7) can be given. 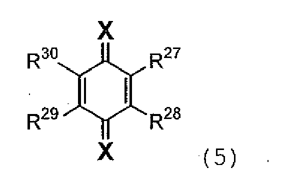 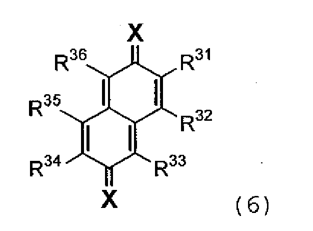 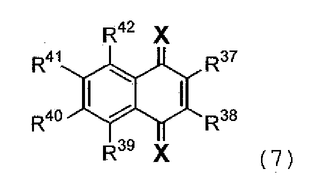 wherein R²⁷ to R⁴² are each a hydrogen atom, a halogen atom, a cyano group, an alkoxy group, a substituted or unsubstituted aryloxy group, an alkyl group, a fluoroalkyl group, an aryl group or a heterocyclic group; provided that at least one of R²⁷ to R³⁰, at least one of R³¹ to R³⁶ or at least one of R³⁷ to R⁴² is a fluorine atom or an aryloxy group having a fluoroalkyl group; X is the same as Xs of the formulas (1) to (3); and preferable examples of R²⁷ to R⁴² are the same as R¹ to R⁴.

The quinone derivative of the invention can be produced by the following method and the like.
For example, in reference to the method of Kallmayer et al. (Pharmazie, 49, 4, 235 (1994)) shown in the scheme 1, an aryloxy benzoquinone derivative can be synthesized by reacting a halogenated benzoquinone derivative with a corresponding phenol and potassium t-butoxide in a DMSO solvent. Various quinone derivatives can be synthesized in reference to the method of Cowan et al. (J. Chem. Soc., Chem. Commun., 286 (1985)) in which the aryloxy benzoquinone derivative is reacted with malononitrile, or the method shown in the scheme 2 in which the aryloxy benzoquinone derivative is reacted with bis(trimethylsilyl)carbodiimide.

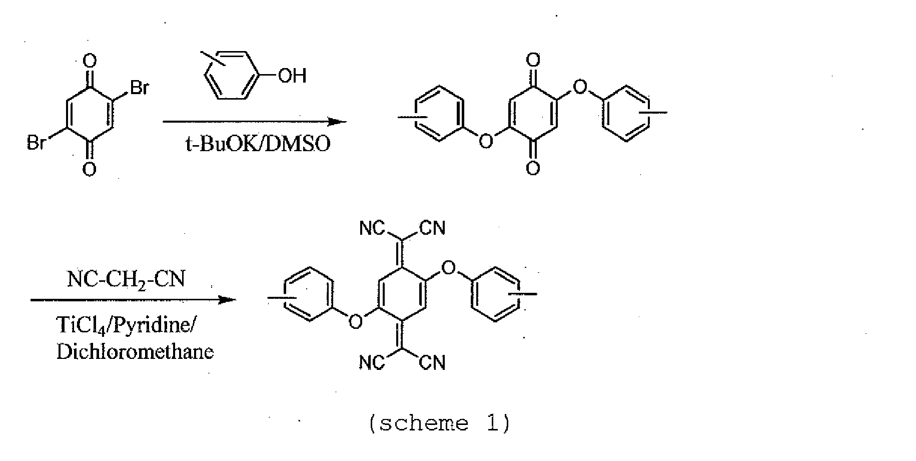

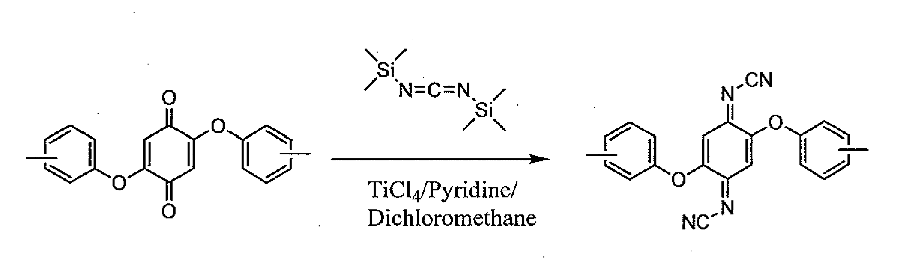

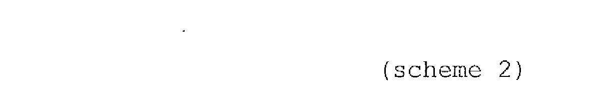

The quinone derivative of the invention has high electron-accepting properties and low crystallinity. This derivative can be used as a material for an organic EL device.

Next, the organic EL device of the invention will be described below.
The organic EL device according to the invention includes a cathode, an anode, and an organic thin film layer provided between these electrodes and formed of one or more layers including an emitting layer.
FIG. 1 is a schematic cross-sectional view showing an example of the organic EL device according to the invention.
In the organic EL device 1, an anode 10, a hole-injecting layer 20, a hole-transporting layer 30, an emitting layer 40, an electron-transporting layer 50, and a cathode 60 are stacked on a substrate (not shown) in this order. In this device, the organic thin layer has a stacked structure of the hole-injecting layer 20, the hole-transporting layer 30, the emitting layer 40, and the electron-transporting layer 50.

In the organic EL device of the invention, at least one of the layers constituting the organic thin layers contains the quinone derivative represented by any one of the above-mentioned formulas (1) to (3). This leads to a lowered driving voltage and a prolonged lifetime of an organic EL device.
The content of the derivative in the layer containing the quinone derivative of the invention is preferably 1 to 100 mol%.

In the organic EL device of the invention, it is preferred that a layer which is present in a region (hole-transporting region) between the anode 10 and the emitting layer 40, specifically, the hole-injecting layer 20 or the hole-transporting layer 30, contain the quinone derivative of the invention. In the device having both the hole-injecting layer 20 and the hole-transporting layer 30 like the embodiment, it is preferred that the hole-injecting layer 20 nearer the anode contain the quinone derivative of the invention.

When the quinone derivative represented by the formulas (1) to (3) are used in the layer present in the hole-transporting region, the quinone derivative of the invention may form the hole-injecting layer or the hole-transporting layer singly or in combination with other materials.
For example, when the the quinone derivative represented by the formulas (1) to (3) and an aromatic amine derivative are mixed to form the hole-injecting layer or the hole-transporting layer, it is preferable to use a phenylenediamine compound represented by the formula (4).

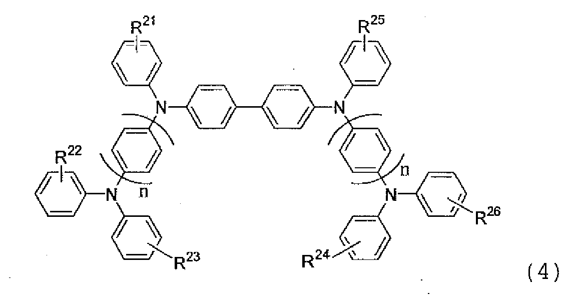

wherein R²¹ to R²⁶ are a hydrogen atom, a halogen atom, a trifluoromethyl group, an alkyl group, an aryl group, or a heterocycle; R²¹ to R²⁶ may form a naphthalene skeleton, a carbazole skeleton, or a fluorene skeleton with a phenyl group bonded; and n represents 1 or 2.
If the above phenylenediamine compound is contained in combination, uniformity, heat resistance, or carrier-injection properties of the film may be improved as compared with a case where the material for an organic EL device of the invention is contained singly.

In the formula (4), fluorine is preferable as the halogen atom represented by R²¹ to R²⁶.

As the alkyl group represented by R²¹ to R²⁶, methyl, isopropyl, tert-butyl, and cyclohexyl are preferred, for example.

As the aryl group represented by R²¹ to R²⁶, phenyl, naphthyl, and fluorenyl are preferable.

As the heterocycle represented by R²¹ to R²⁶, pyridine and pyrazine are preferable, for example.

R²¹ to R²⁶ may form a naphthalene skeleton, a carbazole skeleton, or a fluorene skeleton with a phenyl group bonded.

The content of the compound represented by the formula (4) in the hole-transporting layer or the hole-injecting layer is preferably 0.1 to 99 mol%.
Preferred examples of the compound (4) are given below.

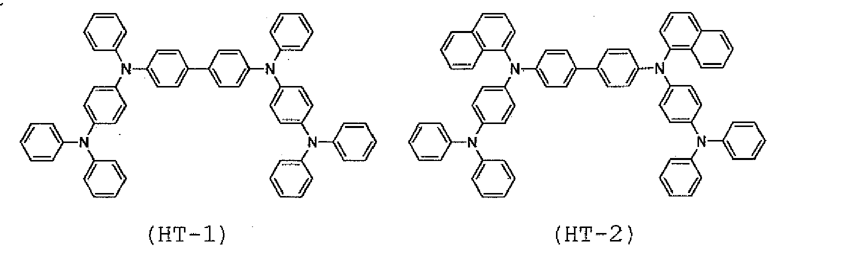

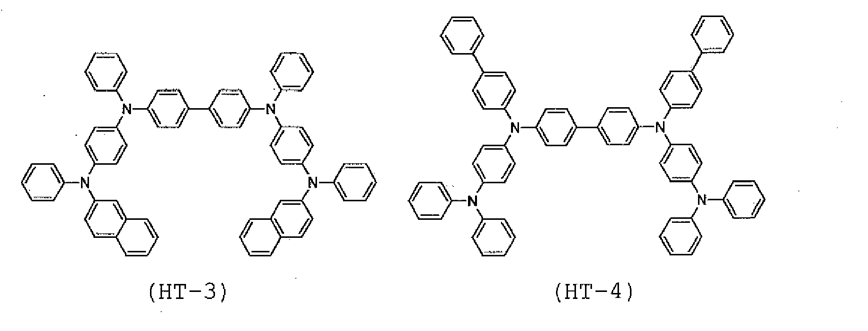

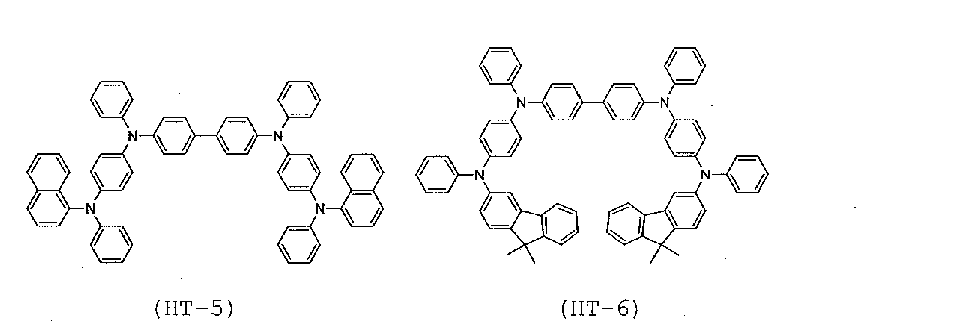

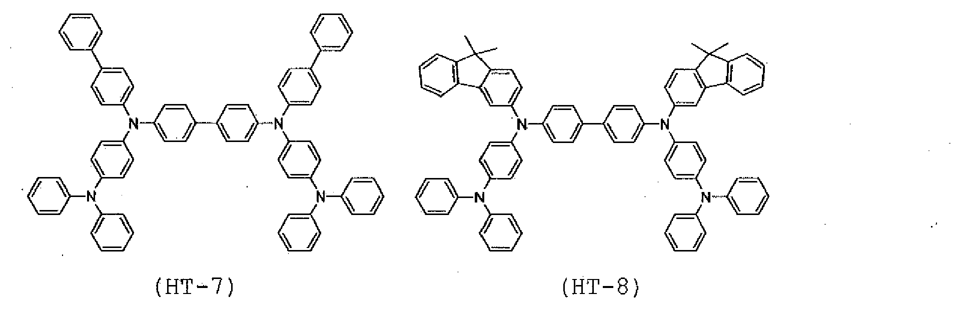

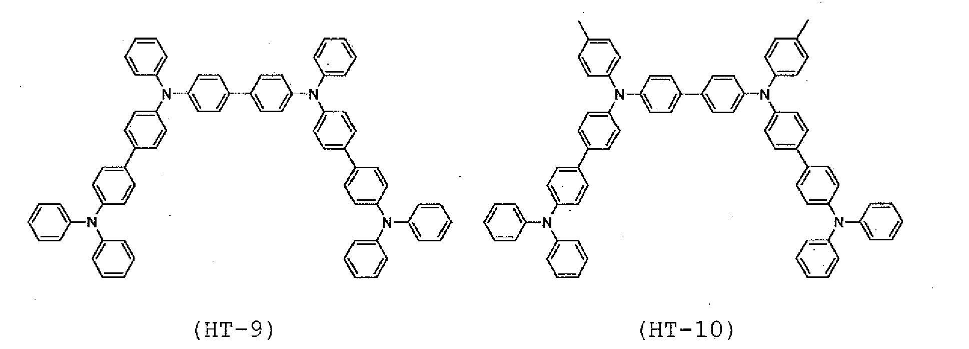

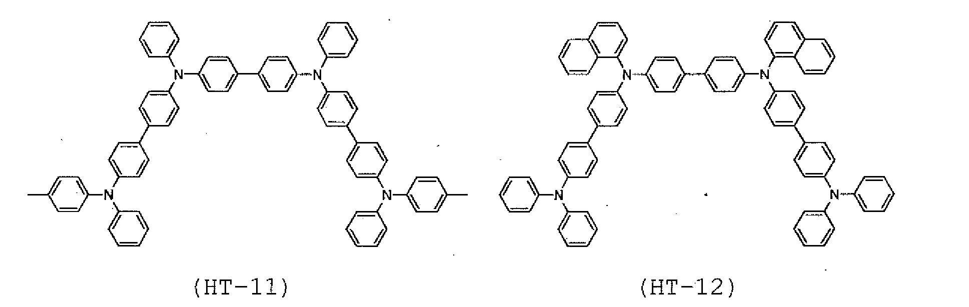

The structure of the organic EL device of the invention is not limited to the embodiment described above. For example, the organic EL device of the invention may have structures (1) to (15) shown below.
(1) Anode/emitting layer/cathode
(2) Anode/hole-transporting layer/emitting layer/cathode
(3) Anode/emitting layer/hole-transporting layer/cathode
(4) Anode/hole-transporting layer/emitting layer/electron-transporting layer/cathode
(5) Anode/hole-transporting layer/emitting layer/adhesion-improving layer/cathode
(6) Anode/hole-injecting layer/hole-transporting layer/emitting layer/electron-transporting layer/cathode (FIG. 1)
(7) Anode/hole-transporting layer/emitting layer/electron-transporting layer/electron-injecting layer/cathode
(8) Anode/hole-injecting layer/hole-transporting layer/emitting layer/electron-transporting layer/electron-injecting layer/cathode
(9) Anode/insulative layer/hole-transporting layer/emitting layer/electron-transporting layer/cathode
(10) Anode/hole-transporting layer/emitting layer/electron-transporting layer/insulative layer/cathode
(11) Anode/inorganic semiconductor layer/insulative layer/hole-transporting layer/emitting layer/insulative layer/cathode
(12) Anode/insulative layer/hole-transporting layer/emitting layer/electron-transporting layer/insulative layer/cathode
(13) Anode/hole-injecting layer/hole-transporting layer/emitting layer/electron-transporting layer/insulative layer/cathode
(14) Anode/insulative layer/hole-injecting layer/hole-transporting layer/emitting layer/electron-transporting layer/electron-injecting layer/cathode
(15) Anode/insulative layer/hole-injecting layer/hole-transporting layer/emitting layer/electron-transporting layer/electron-injecting layer/insulative layer/cathode
Among these, the structures (4), (6), (7), (8), (12), (13) and (15) are generally preferably used.
Each member constituting the organic EL device of the invention will be described below.

### [Substrate]

If an organic EL device is of under surface emission type or bottom emission type where light is outcoupled through a substrate, the organic EL device of the invention is formed on a transparent substrate. The transparent substrate is a substrate for supporting the organic EL device, and is preferably a flat and smooth substrate having a transmittance of 50% or more to light rays within visible ranges of 400 to 700 nm.
Specific examples thereof include glass plates and polymer plates. Examples of the glass plate include soda-lime glass, barium/strontium-containing glass, lead glass, aluminosilicate glass, borosilicate glass, barium borosilicate glass, and quartz. Examples of the polymer plate include polycarbonate, acrylic polymer, polyethylene terephthalate, polyethersulfide, and polysulfone. The substrate may be a TFT substrate in which TFT for driving is formed.

### [Anode]

The anode of the organic thin film EL device plays a role for injecting holes into its hole-transporting layer or emitting layer. When transparency is required for the anode, indium tin oxide alloy (ITO), tin oxide (NESA), zinc tin oxide alloy (IZO), gold, silver, platinum, copper, and the like may be used as the material for the anode. When a reflective electrode which does not require transparency is used, a metal such as aluminum, molybdenum, chromium, and nickel or alloys thereof may also be used.
Although these materials may be used individually, alloys thereof or materials wherein another element is added to the materials can be selected for use.
The anode can be formed by forming these electrode materials into a thin film by vapor deposition, sputtering or the like.
In the case where emission from the emitting layer is taken out through the anode, the transmittance of the anode to the emission is preferably more than 10%. The sheet resistance of the anode is preferably several hundreds Ω/□ or less. The film thickness of the anode, which varies depending upon the material thereof, is usually from 10 nm to 1 µm, preferably from 10 to 200 nm.

### [Emitting layer]

The emitting layer of the organic EL device has the following functions (1), (2) and (3) in combination.
(1) Injecting function: function of allowing injection of holes from anode or hole-injecting layer and injection of electrons from cathode or electron-injecting layer upon application of electric field
(2) Transporting function: function of moving injected carriers (electrons and holes) due to the force of an electric field
(3) Emitting function: function of allowing electrons and holes to recombine to emit light

Note that electrons and holes may be injected into the emitting layer with different degrees, or the transportation capabilities indicated by the mobility of holes and electrons may differ. It is preferable that the emitting layer move either electrons or holes.

As the method of forming the emitting layer, a known method such as deposition, spin coating, or an LB method may be applied. It is preferable that the emitting layer be a molecular deposition film. The term "molecular deposition film" refers to a thin film formed by depositing a vapor-phase material compound or a film formed by solidifying a solution-state or liquid-phase material compound. The molecular deposition film is distinguished from a thin film (molecular accumulation film) formed using the LB method by the difference in aggregation structure or higher order structure or the difference in function due to the difference in structure.
The emitting layer may also be formed by dissolving a binder such as a resin and a material compound in a solvent to obtain a solution, and forming a thin film from the solution by spin coating or the like, as disclosed in JP-A-57-51781.

In the invention, if need arises, known emitting materials other than the emitting materials formed of the novel compound of the invention may be contained in the emitting layer insofar as the object of the invention is not impaired. An emitting layer containing other known emitting materials may be stacked on the emitting layer containing the emitting materials formed of the novel compound of the invention.

As the emitting material or the doping material used for the emitting layer, anthracene, naphthalene, phenanthrene, pyrene, tetracene, coronene, chrysene, fluorescein, perylene, phthaloperylene, naphthaloperylene, perynone, phthaloperynone, naphthaloperynone, diphenylbutadiene, tetraphenylbutadiene, coumarin, oxadiazole, aldazine, bisbenzoxazoline, bisstyryl, pyrazine, cyclopentadiene, a quinoline metal complex, an aminoquinoline metal complex, a benzoquinoline metal complex, imine, diphenyl ethylene, vinylanthracene, diaminocarbazol, pyran, thiopyran, polymethine, merocyanine, an imidazole chelate oxanoid compound, quinacridone, rubrene, a fluorescent pigment and like can be given. Note that the emitting material and the doping material are not limited to these compounds.

As the host material for use in the emitting layer, the compounds represented by the following formulas (i) to (ix) are preferred.
Asymmetrical anthracene represented by the following formula (i) 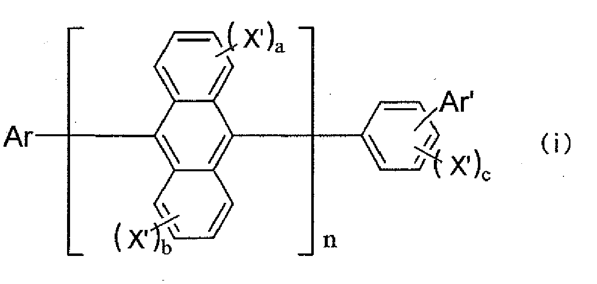 wherein Ar is a substituted or unsubstituted condensed aromatic group having 10 to 50 nucleus carbon atoms,
Ar' is a substituted or unsubstituted aromatic group having 6 to 50 nucleus carbon atoms,
X' is a substituted or unsubstituted aromatic group having 6 to 50 nucleus carbon atoms, a substituted or unsubstituted aromatic heterocyclic group having 5 to 50 nucleus atoms, a substituted or unsubstituted alkyl group having 1 to 50 carbon atoms, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 nucleus atoms, a substituted or unsubstituted arylthio group having 5 to 50 nucleus atoms, a substituted or unsubstituted alkoxycarbonyl group having 1 to 50 carbon atoms, a carboxyl group, a halogen atom, a cyano group, a nitro group or a hydroxyl group.
a, b and c are each an integer of 0 to 4.
n is an integer of 1 to 3. When n is two or more, the groups in [] may be the same or different.

Asymmetrical monoanthracene derivatives represented by the following formula (ii) 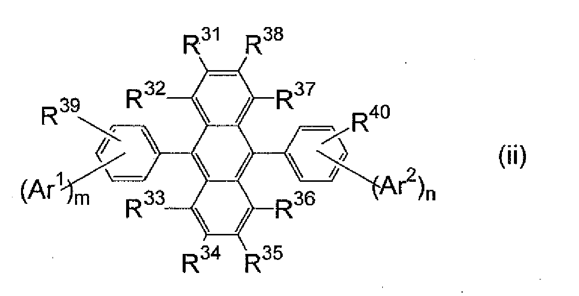 wherein Ar¹ and Ar² are independently a substituted or unsubstituted aromatic ring group having 6 to 50 nucleus carbon atoms, and m and n are each an integer of 1 to 4, provided that in the case where m=n=1 and Ar¹ and Ar² are symmetrically bonded to the benzene rings, Ar¹ and Ar² are not the same, and in the case where m or n is an integer of 2 to 4, m is different from n.
R³¹ to R⁴⁰ are independently a hydrogen atom, a substituted or unsubstituted aromatic ring group having 6 to 50 nucleus carbon atoms, a substituted or unsubstituted aromatic heterocyclic group having 5 to 50 nucleus atoms, a substituted or unsubstituted alkyl group having 1 to 50 carbon atoms, a substituted or unsubstituted cycloalkyl group, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 nucleus atoms, a substituted or unsubstituted arylthio group having 5 to 50 nucleus atoms, a substituted or unsubstituted alkoxycarbonyl group having 1 to 50 carbon atoms, a substituted or unsubstituted silyl group, a carboxyl group, a halogen atom, a cyano group, a nitro group or a hydroxyl group.

Asymmetrical pyrene derivatives represented by the following formula (iii) 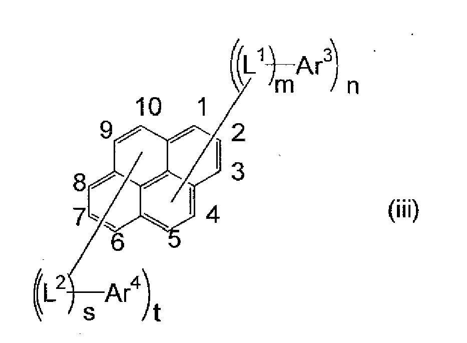 wherein Ar³ and Ar⁴ are each a substituted or unsubstituted aromatic group having 6 to 50 nucleus carbon atoms;
L¹ and L² are each a substituted or unsubstituted phenylene group, a substituted or unsubstituted naphthalenylene group, a substituted or unsubstituted fluolenylene group, or a substituted or unsubstituted dibenzosilolylene group;
m is an integer of 0 to 2, n is an integer of 1 to 4, s is an integer of 0 to 2, and t is an integer of 0 to 4;
L¹ or Ar³ bonds at any one position of 1 to 5 of the pyrene, and L² or Ar⁴ bonds at any one position of 6 to 10 of the pyrene;
provided that when n+t is an even number, Ar³, Ar⁴, L¹ and L² satisfy the following (1) and (2):
(1) Ar³≠Ar⁴ and/or L¹≠L² where ≠ means these substituents are groups having different structures from each other.
(2) when Ar³=Ar⁴ and L¹=L²,
   (2-1) m≠s and/or n≠t, or
   (2-2) when m=s and n=t,
      (2-2-1) L¹ and L², or the pyrene each bond to Ar³ and Ar⁴ at different positions, or
      (2-2-2) when L¹ and L², or the pyrene each bond to Ar³ and Ar⁴ at the same positions, the pyrene is neither substituted by L¹ and L², or Ar³ and Ar⁴ at 1 and 6 positions, nor 2 and 7 positions.

Asymmetrical anthracene represented by the following formula (iv) 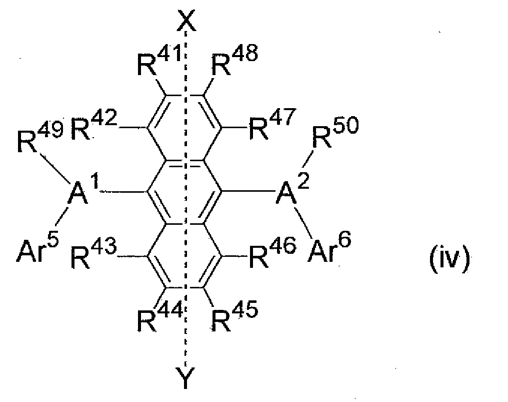 wherein A¹ and A² are independently a substituted or unsubstituted condensed aromatic ring group having 10 to 20 nucleus carbon atoms,
Ar⁵ and Ar⁶ are independently a hydrogen atom or a substituted or unsubstituted aromatic ring group with 6 to 50 nucleus carbon atoms,
R⁴¹ to R⁵⁰ are independently a hydrogen atom or a substituted or unsubstituted aromatic ring group having 6 to 50 nucleus carbon atoms, a substituted or unsubstituted aromatic heterocyclic group having 5 to 50 nucleus atoms, a substituted or unsubstituted alkyl group having 1 to 50 carbon atoms, a substituted or unsubstituted cycloalkyl group, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 nucleus atoms, a substituted or unsubstituted arylthio group having 5 to 50 nucleus atoms, a substituted or unsubstituted alkoxycarbonyl group having 1 to 50 carbon atoms, a substituted or unsubstituted silyl group, a carboxyl group, a halogen atom, a cyano group, a nitro group or a hydroxyl group, and each of Ar⁵, Ar⁶, R⁴⁹ and R⁵⁰ may be plural, and adjacent groups thereof may form a saturated or unsaturated ring structure, provided that groups do not symmetrically bond to 9 and 10 positions of the central anthracene with respect to X-Y axis.

Anthracene derivative represented by the following formula (v) 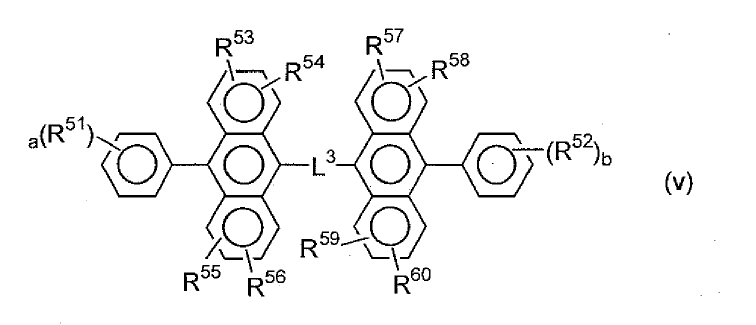 wherein R⁵¹ to R⁶⁰ are independently a hydrogen atom, an alkyl group, a cycloalkyl group, an aryl group which may be substituted, an alkoxy group, an aryloxy group, an alkylamino group, an alkenyl group, an arylamino group or a heterocyclic group which may be substituted; a and b are each an integer of 1 to 5; when they are 2 or more, R⁵¹s or R⁵²s may be the same or different, or R⁵¹s or R⁵²s may be bonded together to form a ring; R⁵³ and R⁵⁴, R⁵⁵ and R⁵⁶, R⁵⁷ and R⁵⁸, or R⁵⁹ and R⁶⁰ may be bonded together to form a ring; and L³ is a single bond, -O-, -S-, -N(R)- (R is an alkyl group or a substituted or unsubstituted aryl group), an alkylene group or an arylene group.

Anthracene derivative represented by the following formula (vi) 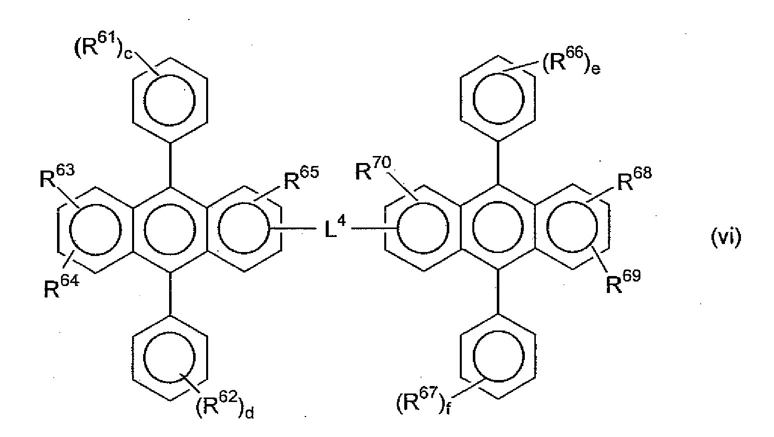 wherein R⁶¹ to R⁷⁰ are independently a hydrogen atom, an alkyl group, a cycloalkyl group, an aryl group, an alkoxy group, an aryloxy group, an alkylamino group, an arylamino group or a heterocyclic group which may be substituted; c, d, e and f are each an integer of 1 to 5; when they are 2 or more, R⁶¹s, R⁶²s, R⁶⁶s or R⁶⁷s may be the same or different, R⁶¹s, R⁶²s, R⁶⁶s or R⁶⁷s may be bonded together to form a ring, or R⁶³ and R⁶⁴, or R⁶⁸ and R⁶⁹ may be bonded together to form a ring; and L⁴ is a single bond, -O-, -S-, -N(R)- (R is an alkyl group or a substituted or unsubstituted aryl group), an alkylene group or an arylene group.

Spirofluorene derivatives represented by the following formula (vii) 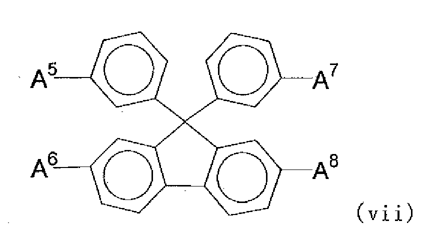 wherein A⁵ to A⁸ are each independently a substituted or unsubstituted biphenyl group or a substituted or unsubstituted naphthyl group.

Condensed ring-containing compounds represented by the following formula (viii) 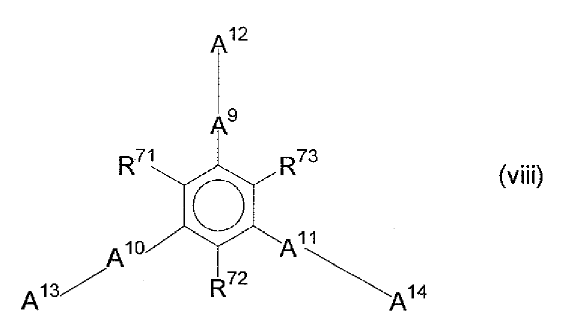 wherein A⁹ to A¹⁴ are the same as the above-described ones and R⁷¹ to R⁷³ are independently a hydrogen atom, alkyl group having 1 to 6 carbon atoms, cycloalkyl group having 3 to 6 carbon atoms, alkoxy group having 1 to 6 carbon atoms, aryloxy group having 5 to 18 carbon atoms, aralkyloxy group having 7 to 18 carbon atoms, arylamino group having 5 to 16 carbon atoms, nitro group, cyano group, ester group having 1 to 6 carbon atoms, or a halogen atom, provided that at least one of A⁹ to A¹⁴ is a group having a condensed aromatic ring with three or more rings.

Fluorene compounds represented by the following formula (ix) 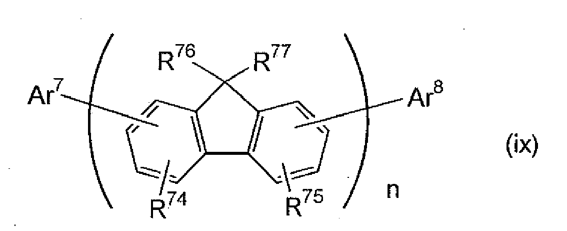 wherein R⁷⁴ and R⁷⁵ are a hydrogen atom, a substituted or unsubstituted alkyl group, substituted or unsubstituted
aralkyl group, substituted or unsubstituted aryl group, substituted or unsubstituted heterocyclic group, substituted amino group, cyano group, or a halogen atom. R⁷⁴s or R⁷⁵s bonded to different fluorene groups may be the same or different, and R⁷⁴ and R⁷⁵ bonded to a single fluorene group may be the same or different. R⁷⁶ and R⁷⁷ are a hydrogen atom, a substituted or unsubstituted alkyl group, substituted or unsubstituted aralkyl group, substituted or unsubstituted aryl group, or substituted or unsubstituted heterocyclic group, provided that R⁷⁶s or R⁷⁷s bonded to different fluorene groups may be the same or different, and R⁷⁶ and R⁷⁷ bonded to a single fluorene group may be the same or different. Ar⁷ and Ar⁸ are a substituted or unsubstituted condensed polycyclic aromatic group with a total number of benzene rings of three or more or a condensed polycyclic heterocyclic group which is bonded to the fluorene group through substituted or unsubstituted carbon and has a total number of benzene rings and heterocyclic rings of three or more, provided that Ar⁷ and Ar⁸ may be the same or different. n is an integer of 1 to 10.

Among the above compounds, the host material is preferably the anthracene derivative, more preferably the monoanthracene derivative, and particularly the asymmetrical anthracene.

Phosphorescent compounds can be used as a dopant of an emitting material.
When using a phosphorescent compound, compounds containing a carbazole ring are preferred for a host material.
A phosphorescent dopant is a compound that can emit light from triplet excitons. The dopant is not limited so long as it can emit light from triplet excitons, but it is preferably a metal complex containing at least one metal selected from the group of Ir, Ru, Pd, Pt, Os and Re. A porphyrin metal complex or an ortho-metalated metal complex is preferable.

The compounds containing a carbazole ring, which are a host suitable for phosphorescence emission, is a compound which allows a phosphorescent compound to emit as a result of energy transfer from its excited state to the phosphorescent compound. A host compound is not limited so long as the compound can transfer its excited energy to a phosphorescent compound and it can be selected depending on purposes. The host compound may contain any heterocyclic ring other than a carbazole ring.

Specific examples of the host compounds include carbazole, triazole, oxazole, oxadiazole, imidazole, polyarylalkane, pyrazoline, pyrazolone, phenylanediamine, arylamine, amino-substituted calcone, styryl anthracene, fluorenone, hydrazone, stilbene and silazane derivatives; aromatic tertiary amine, styrylamine, aromatic dimethylidene and porphyrin compounds; anthraquinodimethane, anthrone, diphenylquinone, thiopyrandioxide, carbodiimide, fluoreniridenemethane and distyrylpyrazine derivatives; heterocyclic tetracarboxylic anhydrides such as naphthaleneperylene; phthalocyanine derivatives; metal complexes of 8-quinolinol derivatives; various metal complex polysilane compounds represented by metal complexes having metalphthalocyanine, benzoxazole or benzothiaole as a ligand; electroconductive macromolecular oligomers such as poly(N-vinylcarbazole) derivatives, aniline copolymers, thiophene oligomers and polythiophene; and macromolecular compounds such as polythiophene, polyphenylene, polyphenylenevinylene and polyfluorene derivatives. Host compounds can be used individually or as a combination of two or more kinds.
Specific compounds shown below can be exemplified.

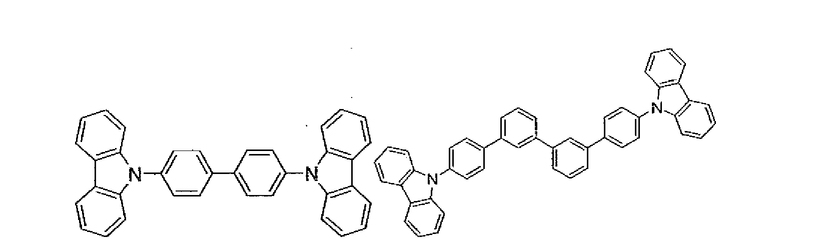

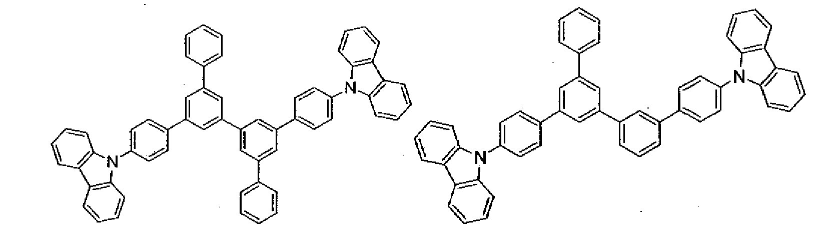

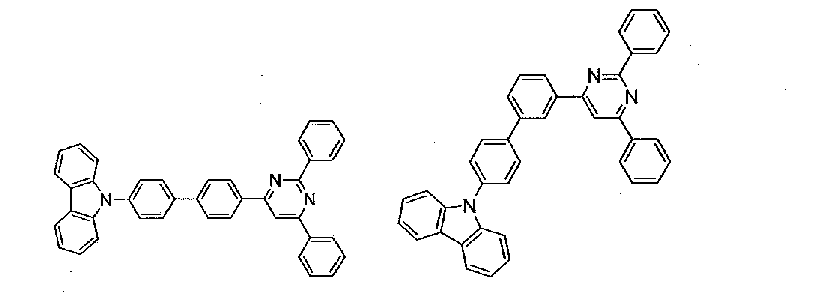

A phosphorescent dopant is a compound that can emit light from triplet excitons. The dopant is not limited so long as it can emit light from triplet excitons, but it is preferably a metal complex containing at least one metal selected from the group of Ir, Ru, Pd, Pt, Os and Re. A porphyrin metal complex or an ortho-metalated metal complex is preferable. As a porphyrin metal complex, a porphyrin platinum complex is preferable. The phosphorescent compounds can be used individually or as a combination of two or more kinds.

There are various ligands forming an ortho-metalated metal complex. Preferable ligands include 2-phenylpyridine, 7,8-benzoquinoline, 2-(2-thienyl)pyridine, 2-(1-naphtyl)pyridine and 2-phenylquinoline derivatives. These derivatives may have substituents, if necessary. Fluorides and derivatives with a trifluoromethyl group introduced are particularly preferable as a blue dopant. As an auxiliary ligand, preferred are ligands other than the above-mentioned ligands, such as acetylacetonate and picric acid may be contained.

The content of a phosphorescent dopant in an emitting layer is not limited and can be properly selected according to purposes; for example, it is 0.1 to 70 mass %, preferably 1 to 30 mass %. When the content of a phosphorescent compound is less than 0.1 mass %, emission may be weak and the advantages thereof may not be sufficiently obtained. When the content exceeds 70 mass %, the phenomenon called concentration quenching may significantly proceed, thereby degrading the device performance.

The emitting layer may contain hole-transporting materials, electron-transporting materials and polymer binders, if necessary.
The thickness of an emitting layer is preferably from 5 to 50 nm, more preferably from 7 to 50 nm and most preferably from 10 to 50 nm. When it is less than 5 nm, the formation of an emitting layer and the adjustment of chromaticity may become difficult. When it exceeds 50 nm, the driving voltage may increase.

### [Hole-transporting/injecting layer]

The hole-transporting layer is a layer for helping the injection of holes into the emitting layer so as to transport holes to an emitting region. The hole mobility thereof is large and the ionization energy thereof is usually as small as 5.5 eV or less. Such a hole-transporting layer is preferably made of a material which can transport holes to the emitting layer at a low electric field intensity. The hole mobility thereof is preferably at least 10⁻⁴ cm²/V·second when an electric field of, e.g., 10⁴ to 10⁶ V/cm is applied.

Specific examples of materials for a hole-transporting layer include triazole derivatives (see USP No. 3,112,197 and others), oxadiazole derivatives (see USP No. 3,189,447 and others), imidazole derivatives (see JP-B-37-16096 and others), polyarylalkane derivatives (see USP Nos. 3,615,402, 3,820,989 and 3,542,544, JP-B-45-555 and 51-10983, JP-A-51-93224, 55-17105, 56-4148, 55-108667, 55-156953 and 56-36656, and others), pyrazoline derivatives and pyrazolone derivatives (see USP Nos. 3,180,729 and 4,278,746, JP-A-55-88064, 55-88065, 49-105537, 55-51086, 56-80051, 56-88141, 57-45545, 54-112637 and 55-74546, and others), phenylene diamine derivatives (see USP No. 3,615,404, JP-B-51-10105, 46-3712 and 47-25336, JP-A-54-53435, 54-110536 and 54-119925, and others), arylamine derivatives (see USP Nos. 3,567,450, 3,180,703, 3,240,597, 3,658,520, 4,232,103, 4,175,961 and 4,012,376, JP-B-49-35702 and 39-27577, JP-A-55-144250, 56-119132 and 56-22437, DE1,110,518, and others), amino-substituted chalcone derivatives (see USP No. 3,526,501, and others), oxazole derivatives (ones disclosed in USP No. 3,257,203, and others), styrylanthracene derivatives (see JP-A-56-46234, and others), fluorenone derivatives (JP-A-54-110837, and others), hydrazone derivatives (see USP Nos. 3,717,462, JP-A-54-59143, 55-52063, 55-52064, 55-46760, 55-85495, 57-11350, 57-148749 and 2-311591, and others), stilbene derivatives (see JP-A-61-210363, 61-228451, 61-14642, 61-72255, 62-47646, 62-36674, 62-10652, 62-30255, 60-93455, 60-94462, 60-174749 and 60-175052, and others), silazane derivatives (USP No. 4,950,950), polysilanes (JP-A-2-204996), aniline copolymers (JP-A-2-282263), and electroconductive high molecular oligomers (in particular thiophene oligomers) disclosed in JP-A-1-211399.

In addition to the hole-transporting layer, in order to help the injection of holes, it is preferred that the hole-injecting layer be provided separately. As the material for the hole-injecting layer, the material of the organic EL of the invention may be used singly or in combination with other materials. As the other materials, the same materials as used for the hole-transporting layer or the compounds exemplified by the above-mentioned formula (4) can be used. The above-mentioned substances can be used as the material of the hole-injecting layer or the hole-transporting layer. The following can also be used: porphyrin compounds (disclosed in JP-A-63-2956965 and others), aromatic tertiary amine compounds and styrylamine compounds (see USP No. 4,127,412, JP-A-53-27033, 54-58445, 54-149634, 54-64299, 55-79450, 55-144250, 56-119132, 61-295558, 61-98353 and 63-295695, and others), and aromatic tertiary amine compounds.

The following can also be given as examples: 4,4'-bis(N-(1-naphthyl)-N-phenylamino)biphenyl, which has in the molecule thereof two condensed aromatic rings, disclosed in USP No. 5,061,569, and 4,4',4"-tris(N-(3-methylphenyl)-N-phenylamino)triphenylamine (MTDATA), wherein three triphenylamine units are linked to each other in a star-burst form, disclosed in JP-A-4-308688.

Inorganic compounds such as p-type Si and p-type SiC as well as aromatic dimethylidene type compounds can also be used as the material of the hole-transporting layer.

The hole-transporting layer can be formed from the above-mentioned compounds by a known method such as vacuum deposition, spin coating, casting or LB technique. The film thickness of the hole-injecting/transporting layer is not particularly limited, and is usually from 5 nm to 5 µm. This hole-injecting layer or the hole-transporting layer may be a single layer made of one or more of the above-mentioned materials, or may be stacked hole-injecting layers or hole-transporting layers made of different compounds, insofar as the compound of the invention is contained.

An organic semiconductor layer is one type of a hole-transporting layer for helping the injection of holes or electrons into an emitting layer, and is preferably a layer having an electric conductivity of 10⁻¹⁰ S/cm or more. As the material of such an organic semiconductor layer, electroconductive oligomers such as thiophene-containing oligomers or arylamine-containing oligomers disclosed in JP-A-8-193191, and electroconductive dendrimers such as arylamine-containing dendrimers may be used.

### [Electron-injecting/transporting layer]

The electron-injecting/transporting layer is a layer which assists injection of electrons into the emitting layer and transports electrons to the emitting region, and exhibits a high electron mobility. An adhesion-improving layer is formed of a material which exhibits excellent adhesion to the cathode.
The thickness of the electron-transporting layer is arbitrarily selected in the range of several nanometers to several micrometers. When the electron-transporting layer has a large thickness, it is preferable that the electron mobility be at least 10⁻⁵ cm²/Vs or more at an applied electric field of 10⁴ to 10⁶ V/cm in order to prevent an increase in voltage.

The material used in the electron-transporting layer is preferably a metal complex of 8-hydroxyquinoline or a derivative thereof. As specific examples of 8-hydroxyquinoline and a metal complex of an 8-hydroxyquinoline derivative, metal chelate oxinoid compounds including a chelate of oxine (8-quinolinol or 8-hydroxyquinoline) can be given.

An electron-transporting compound of the following general formula can be given as the oxadiazole derivative.

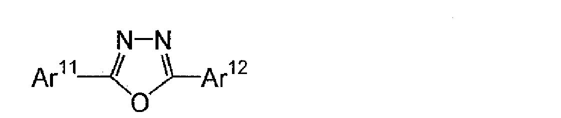

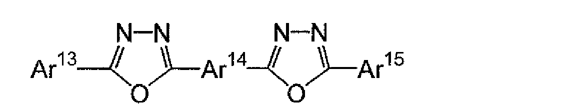

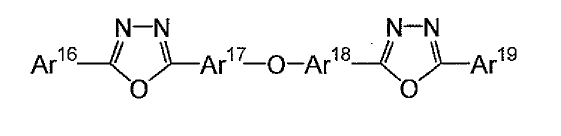

wherein Ar¹¹, Ar¹², Ar¹³, Ar¹⁵, Ar¹⁶, and Ar¹⁹ are independently substituted or unsubstituted aryl groups and may be the same or different. Ar¹⁴, Ar¹⁷, and Ar¹⁸ are independently substituted or unsubstituted arylene groups and may be the same or different.

As examples of the aryl group, a phenyl group, a biphenyl group, an anthranyl group, a perylenyl group, and a pyrenyl group can be given. As examples of the arylene group, a phenylene group, a naphthylene group, a biphenylene group, an anthranylene group, a perylenylene group, a pyrenylene group, and the like can be given. As the substituent, an alkyl group having 1 to 10 carbon atoms, an alkoxy group having 1 to 10 carbon atoms, a cyano group, and the like can be given. The electron-transporting compound is preferably one from which a thin film can be formed.

The following compounds can be given as specific examples of the electron-transporting compound.

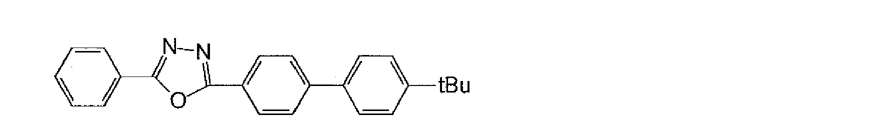

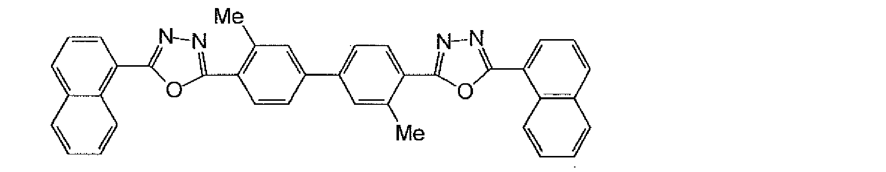

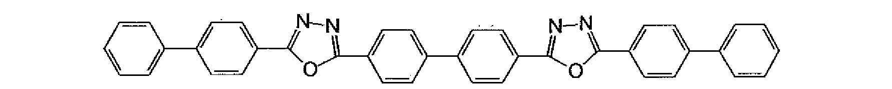

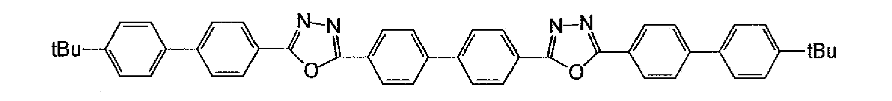

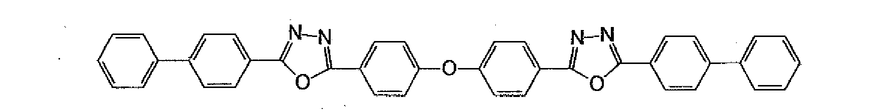

Furthermore, as materials used for the electron-injecting layer and electron-transporting layer, the compounds represented by the following formulas (A) to (F) may be used. 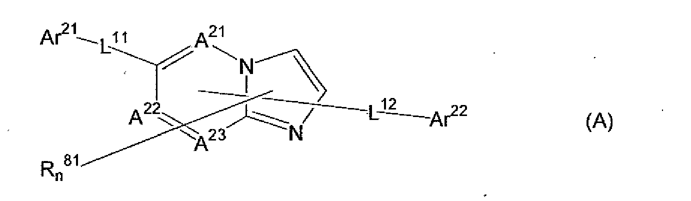 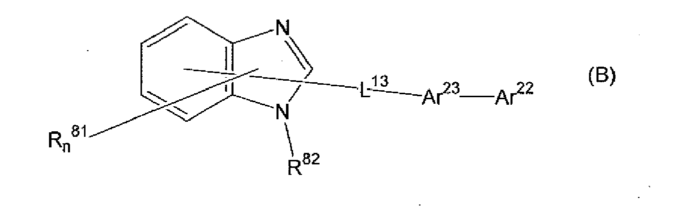 Nitrogen-containing heterocyclic ring derivatives represented by the formulas (A) and (B) wherein A²¹ to A²³ are each independently a nitrogen atom or a carbon atom;
Ar²¹ is a substituted or unsubstituted aryl group having 6 to 60 nucleus carbon atoms or a substituted or unsubstituted heteroaryl group having 3 to 60 nucleus carbon atoms; Ar²² is a hydrogen atom, a substituted or unsubstituted aryl group having 6 to 60 nucleus carbon atoms, a substituted or unsubstituted heteroaryl group having 3 to 60 nucleus carbon atoms, a substituted or unsubstituted alkyl group having 1 to 20 carbon atoms, a substituted or unsubstituted alkoxy group having 1 to 20 carbon atoms, or a divalent group of these; provided that one of Ar²¹ and Ar²² is a substituted or unsubstituted condensed ring group having 10 to 60 nucleus carbon atoms, a substituted or unsubstituted monohetero condensed ring group having 3 to 60 nucleus carbon atoms, or a divalent group of these;
Ar²³ is a substituted or unsubstituted arylene group having 6 to 60 carbon atoms or a substituted or unsubstituted heteroarylene group having 3 to 60 carbon atoms;
L¹¹, L¹², and L¹³ are independently a single bond, a substituted or unsubstituted arylene group having 6 to 60 nucleus carbon atoms, a substituted or unsubstituted heteroarylene group having 3 to 60 nucleus carbon atoms or a substituted or unsubstituted fluorenylene group;
R⁸¹ is a hydrogen atom, a substituted or unsubstituted aryl group having 6 to 60 nucleus carbon atoms, a substituted or unsubstituted heteroaryl group having 3 to 60 nucleus carbon atoms, a substituted or unsubstituted alkyl group having 1 to 20 carbon atoms, or a substituted or unsubstituted alkoxy group having 1 to 20 carbon atoms, and n is an integer of 0 to 5, provided that, when n is an integer of 2 or more, a plurality of R⁸¹s may be the same or different; adjacent R⁸¹s may be bonded to form a carbocyclic aliphatic ring or a carbocyclic aromatic ring; and
R⁸² is a hydrogen atom, a substituted or unsubstituted aryl group having 6 to 60 nucleus carbon atoms, a substituted or unsubstituted heteroaryl group having 3 to 60 nucleus carbon atoms, a substituted or unsubstituted alkyl group having 1 to 20 carbon atoms, a substituted or unsubstituted alkoxy group having 1 to 20 carbon atoms or -L¹¹-Ar²¹-Ar²².

HAr-L¹⁴-Ar²⁴-Ar²⁵ (C)

Nitrogen-containing heterocyclic ring derivatives represented by the formula (C) wherein HAr is a nitrogen-containing heterocyclic ring with 3 to 40 carbon atoms which may have a substituent; L¹⁴ is a single bond, an arylene group with 6 to 60 carbon atoms which may have a substituent, a heteroarylene group with 3 to 60 carbon atoms which may have a substituent or a fluorenylene group which may have a substituent; Ar²⁴ is a divalent aromatic hydrocarbon group with 6 to 60 carbon atoms which may have a substituent; and Ar²⁵ is an aryl group with 6 to 60 carbon atoms which may have a substituent or a heteroaryl group with 3 to 60 carbon atoms which may have a substituent.

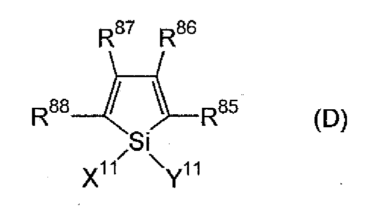

Silacyclopentadiene derivatives represented by the formula (D) wherein X¹¹ and Y¹¹ are independently a saturated or unsaturated hydrocarbon group having 1 to 6 carbon atoms, an alkoxy group, an alkenyloxy group, an alkynyloxy group, a hydroxyl group, a substituted or unsubstituted aryl group, or a substituted or unsubstituted hetero ring, or X¹¹ and Y¹¹ are bonded to form a saturated or unsaturated ring, and R⁸⁵ to R⁸⁸ are independently hydrogen, halogen, a substituted or unsubstituted aryl group having 1 to 6 carbon atoms, an alkoxy group, an aryloxy group, a perfluoroalkyl group, a perfluoroalkoxy group, an amino group, an alkylcarbonyl group, an arylcarbonyl group, an alkoxycarbonyl group, an aryloxycarbonyl group, an azo group, an alkylcarbonyloxy group, an arylcarbonyloxy group, an alkoxycarbonyloxy group, an aryloxycarbonyloxy group, a sulfinyl group, a sulfonyl group, a sulfanyl group, a silyl group, a carbamoyl group, an aryl group, a heterocyclic group, an alkenyl group, an alkynyl group, a nitro group, a formyl group, a nitroso group, a formyloxy group, an isocyano group, a cyanate group, an isocyanate group, a thiocyanate group, an isothiocyanate group, or a cyano group, or adjacent groups of R⁸⁵ to R⁸⁸ from a substituted or unsubstituted condensed ring.

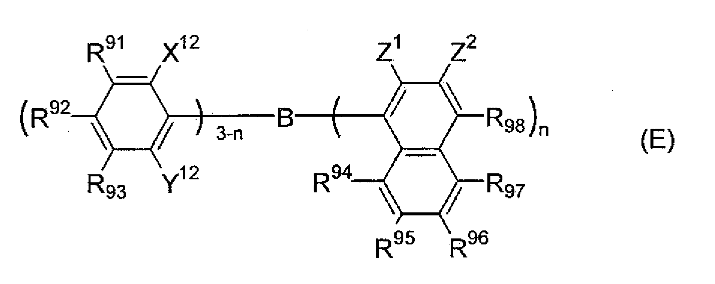

Borane derivatives represented by the formula (E) wherein R⁹¹ to R⁹⁸ and Z² are independently a hydrogen atom, a saturated or unsaturated hydrocarbon group, an aromatic group, a heterocyclic group, a substituted amino group, a substituted boryl group, an alkoxy group, or an aryloxy group, X¹², Y¹², and Z¹ are independently a saturated or unsaturated hydrocarbon group, an aromatic group, a heterocyclic group, a substituted amino group, an alkoxy group, or an aryloxy group, the substituents for Z¹ and Z² may be bonded to form a condensed ring, n is an integer of 1 to 3, provided that the Z¹s may differ when n is 2 or more, and a case in which n is 1, X¹², Y¹², and R⁹² are methyl groups, and R⁹⁸ is a hydrogen atom or a substituted boryl group, and a case in which n is 3 and Z¹ is a methyl group are excluded.

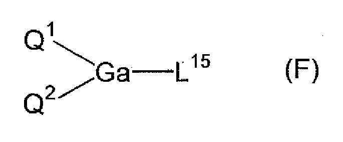

wherein Q¹ and Q² are independently ligands represented by the following formula (G) and L¹⁵ is a halogen atom, a substituted or unsubstituted alkyl group, a substituted or unsubstituted cycloalkyl group, a substituted or unsubstituted aryl group, a substituted or unsubstituted heterocyclic group, -OR' (R' is a hydrogen atom, a substituted or unsubstituted alkyl group, a substituted or unsubstituted cycloalkyl group, a substituted or unsubstituted aryl group, or a substituted or unsubstituted heterocyclic group) or a ligand represented by -O-Ga-Q³(Q⁴) (Q³ and Q⁴ have the same meanings as Q¹ and Q²).

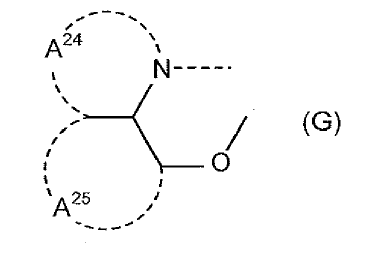

wherein rings A²⁴ and A²⁵ are each a 6-membered aryl ring structure which may have a substituent, and are condensed to each other.

The metal complexes have the strong nature of an n-type semiconductor and large ability of injecting electrons. Further the energy generated at the time of forming a complex is small so that a metal is then strongly bonded to ligands in the complex formed and the fluorescent quantum efficiency becomes large as the emitting material

Specific examples of the substituents for the rings A²⁴ and A²⁵ forming the ligand of the formula G include halogen atoms such as chlorine, bromine, iodine, and fluorine, substituted or unsubstituted alkyl groups such as a methyl group, ethyl group, propyl group, butyl group, sec-butyl group, tert-butyl group, pentyl group, hexyl group, heptyl group, octyl group, stearyl group, and trichloromethyl group, substituted or unsubstituted aryl groups such as a phenyl group, naphthyl group, 3-methylphenyl group, 3-methoxyphenyl group, 3-fluorophenyl group, 3-trichloromethylphenyl group, 3-trifluoromethylphenyl group, and 3-nitrophenyl group, substituted or unsubstituted alkoxy groups such as a methoxy group, n-butoxy group, tert-butoxy group, trichloromethoxy group, trifluoroethoxy group, pentafluoropropoxy group, 2,2,3,3-tetrafluoropropoxy group, 1,1,1,3,3,3-hexafluoro-2-propoxy group, and 6-(perfluoroethyl)hexyloxy group, substituted or unsubstituted aryloxy groups such as a phenoxy group, p-nitrophenoxy group, p-tert-butylphenoxy group, 3-fluorophenoxy group, pentafluorophenyl group, and 3-trifluoromethylphenoxy group, substituted or unsubstituted alkylthio groups such as a methylthio group, ethylthio group, tert-butylthio group, hexylthio group, octylthio group, and trifluoromethylthio group, substituted or unsubstituted arylthio groups such as a phenylthio group, p-nitrophenylthio group, p-tert-butylphenylthio group, 3-fluorophenylthio group, pentafluorophenylthio group, and 3-trifluoromethylphenylthio group, a cyano group, a nitro group, an amino group, mono- or di-substituted amino groups such as a methylamino group, diethylamino group, ethylamino group, diethylamino group, dipropylamino group, dibutylamino group, and diphenylamino group, acylamino groups such as a bis(acetoxymethyl)amino group, bis(acetoxyethyl)amino group, bis(acetoxypropyl)amino group, and bis(acetoxybutyl)amino group, a hydroxyl group, a siloxy group, an acyl group, and a substituted or unsubstituted carbamoyl groups such as a carbamoyl group, a methylcarbamoyl group, dimethylcarbamoyl group, ethylcarbamoyl group, diethylcarbamoyl group, propylcarbamoyl group, butylcarbamoyl group, and phenylcarbamoyl group, a carboxylic acid group, a sulfonic acid group, an imide group, cycloalkyl groups such as a cyclopentane group and a cyclohexyl group, aryl groups such as a phenyl group, naphthyl group, biphenyl group, anthryl group, phenanthryl group, fluorenyl group, and pyrenyl group, heterocyclic groups such as a pyridinyl group, pyrazinyl group, pyrimidinyl group, pyridazinyl group, triazinyl group, indolinyl group, quinolinyl group, acridinyl group, pyrrolidinyl group, dioxanyl group, piperidinyl group, morpholidinyl group, piperazinyl group, carbazolyl group, furanyl group, thiophenyl group, oxazolyl group, oxadiazolyl group, benzooxazolyl group, thiazolyl group, thiadiazolyl group, benzothiazolyl group, triazolyl group, imidazolyl group, benzimidazolyl group, and the like. The above substituents may be bonded to form a six-membered aryl ring or heterocyclic ring.

A preferred embodiment of the invention is a device containing a reducing dopant in an interfacial region between its electron transferring region or cathode and organic layer. The reducing dopant is defined as a substance which can reduce an electron transferring compound. Accordingly, various substances which have given reducing properties can be used. For example, at least one substance can be preferably used which is selected from the group consisting of alkali metals, alkaline earth metals, rare earth metals, alkali metal oxides, alkali metal halides, alkaline earth metal oxides, alkaline earth metal halides, rare earth metal oxides, rare earth metal halides, alkali metal organic complexes, alkaline earth metal organic complexes, and rare earth metal organic complexes.

More specific examples of the preferred reducing dopants include at least one alkali metal selected from the group consisting of Li (work function: 2.9 eV), Na (work function: 2.36 eV), K (work function: 2.28 eV), Rb (work function: 2.16 eV) and Cs (work function: 1.95 eV), and at least one alkaline earth metal selected from the group consisting of Ca (work function: 2.9 eV), Sr (work function: 2.0 to 2.5 eV), and Ba (work function: 2.52 eV). Metals having a work function of 2.9 eV or less are particularly preferred.
Among these, a more preferable reducing dopant is at least one alkali metal selected from the group consisting of K, Rb and Cs. Even more preferable is Rb or Cs. Most preferable is Cs.

These alkali metals are particularly high in reducing ability. Thus, the addition of a relatively small amount thereof to an electron-injecting zone improves the luminance of the organic EL device and make the lifetime thereof long. As a reducing agent having a work function of 2.9 eV or less, combinations of two or more alkali metals are preferable, particularly combinations including Cs, such as Cs and Na, Cs and K, Cs and Rb, or Cs, Na and K are preferable.
The combination containing Cs makes it possible to exhibit the reducing ability efficiently. The luminance of the organic EL device can be improved and the lifetime thereof can be made long by the addition thereof to its electron-injecting zone.

In the invention, an electron-injecting layer made of an insulator or a semiconductor may further be provided between a cathode and an organic layer. By providing the layer, current leakage can be effectively prevented to improve the injection of electrons.

As the insulator, at least one metal compound selected from the group consisting of alkali metal calcogenides, alkaline earth metal calcogenides, halides of alkali metals and halides of alkaline earth metals can be preferably used. When the electron-injecting layer is formed of the alkali metal calcogenide or the like, the injection of electrons can be preferably further improved.

Specifically preferable alkali metal calcogenides include Li₂O, LiO, Na₂S, Na₂Se and NaO and preferable alkaline earth metal calcogenides include CaO, BaO, SrO, BeO, BaS and CaSe. Preferable halides of alkali metals include LiF, NaF, KF, LiCl, KCl and NaCl. Preferable halides of alkaline earth metals include fluorides such as CaF₂, BaF₂, SrF₂, MgF₂ and BeF₂ and halides other than fluorides.

Semiconductors forming an electron-transporting layer include one or combinations of two or more of oxides, nitrides, and oxidized nitrides containing at least one element of Ba, Ca, Sr, Yb, Al, Ga, In, Li, Na, Cd, Mg, Si, Ta, Sb and Zn.
An inorganic compound forming an electron-transporting layer is preferably a microcrystalline or amorphous insulating thin film. When the electron-transporting layer is formed of the insulating thin films, more uniformed thin film is formed, whereby pixel defects such as a dark spot are decreased.

Examples of such an inorganic compound include the above-mentioned alkali metal calcogenides, alkaline earth metal calcogenides, halides of alkali metals, and halides of alkaline earth metals.

### [Cathode]

For the cathode, the following may be used: an electrode substance made of a metal, an alloy or an electroconductive compound, or a mixture thereof which has a small work function (4 eV or less). Specific examples of the electrode substance include sodium, sodium-potassium alloy, magnesium, lithium, magnesium/silver alloy, aluminum/aluminum oxide, aluminum/lithium alloy, indium, and rare earth metals.
This cathode can be formed by making the electrode substances into a thin film by vapor deposition, sputtering or some other method.

In the case where emission from the emitting layer is outcoupled through the cathode, it is preferred to make the transmittance of the cathode to the emission larger than 10%.
The sheet resistance of the cathode is preferably several hundreds Ω/□ or less, and the film thickness thereof is usually from 10 nm to 1 µm, preferably from 50 to 200 nm.

### [Insulative layer]

In the organic EL device, pixel defects based on leakage or a short circuit are easily generated since an electric field is applied to the super thin film. In order to prevent this, it is preferred to insert an insulator thin layer between the pair of electrodes.
Examples of the material used in the insulative layer include aluminum oxide, lithium fluoride, lithium oxide, cesium fluoride, cesium oxide, magnesium oxide, magnesium fluoride, calcium oxide, calcium fluoride, cesium fluoride, cesium carbonate, aluminum nitride, titanium oxide, silicon oxide, germanium oxide, silicon nitride, boron nitride, molybdenum oxide, ruthenium oxide, and vanadium oxide.
A mixture or laminate thereof may be used.

### [Example of fabricating organic EL device]

Using the above-mentioned materials, an organic EL device can be fabricated by forming an anode, a hole-injecting layer, a hole-transporting layer, an emitting layer, an electron-injecting layer or the like, followed by formation of a cathode. The organic EL device can be fabricated in the order reverse to the above, i.e., the order from a cathode to an anode.

An example of the fabrication of the organic EL device will be described below which has a structure wherein the following are successively formed on a transparent substrate: anode/hole-injecting layer/hole-transporting layer/emitting layer/electron-transporting layer/cathode.
First, a thin film made of an anode material is formed into a thickness of 1 µm or less, preferably 10 to 200 nm on an appropriate transparent substrate by vapor deposition, sputtering or some other method, thereby forming an anode.

Next, a hole-injecting layer and a hole-transporting layer are formed on this anode. As described above, these layers can be formed by vacuum deposition, spin coating, casting, LB technique, or some other method. Vacuum deposition is preferred since a homogenous film is easily obtained and pinholes are not easily generated.

In the case where the hole-injecting layer and the hole-transporting layer are formed by vacuum deposition, conditions for the deposition vary depending upon the compound used, the desired crystal structure or recombining structure of the hole-transporting layer, and others. In general, the conditions are preferably selected from the following: deposition source temperature of 50 to 450°C, vacuum degree of 10⁻⁷ to 10⁻³ torr, vapor deposition rate of 0.01 to 50 nm/second, substrate temperature of -50 to 300°C, and film thickness of 5 nm to 5 µm.

Next, an emitting layer is formed on the hole-transporting layer. The emitting layer can also be formed by making a desired organic luminescent material into a thin film by vacuum deposition, sputtering, spin coating, casting or some other method. Vacuum deposition is preferred since a homogenous film is easily obtained and pinholes are not easily generated. In the case where the emitting layer is formed by vacuum deposition, conditions for the deposition, which vary depending on a compound used, can be generally selected from conditions similar to those for the hole-transporting layer.

Next, an electron-transporting layer is formed on this emitting layer. Like the hole-transporting layer and the emitting layer, the layer is preferably formed by vacuum deposition because a homogenous film is required. Conditions for the deposition can be selected from conditions similar to those for the hole-transporting layer and the emitting layer.

Lastly, a cathode is stacked thereon to obtain an organic EL device.
The cathode is made of a metal, and vapor deposition or sputtering may be used. However, vacuum deposition is preferred in order to protect underlying organic layers from being damaged when the cathode film is formed.
For the organic EL device fabrication that has been described above, it is preferred that the formation from the anode to the cathode is continuously carried out, using only one vacuuming operation.

The method for forming each of the layers in the organic EL device of the invention is not particularly limited. Specifically the layers can be formed by a known method, such as vacuum deposition, molecular beam deposition (MBE method), or coating method such as dipping, spin coating, casting, bar coating and roll coating using a solution obtained by dissolving materials in a solvent.

The film thickness of each of the organic layers in the organic EL device of the invention is not particularly limited. In general, defects such as pinholes are easily generated when the film thickness is too small. Conversely, when the film thickness is too large, a high applied voltage becomes necessary, leading to low efficiency. Usually, the film thickness is preferably in the range of several nanometers to one micrometer.

The organic EL device emits light when applying a voltage between electrodes. If a DC voltage is applied to the organic EL device, emission can be observed when the polarities of the anode and the cathode are positive and negative, respectively, and a DC voltage of 5 to 40 V is applied. When a voltage with an opposite polarity is applied, no electric current flows and hence, emission does not occur. If an AC voltage is applied, uniform emission can be observed only when the cathode and the anode have a positive polarity and a negative polarity, respectively. The waveform of the AC applied may be arbitrary. The waveform of the AC applied may be arbitrary.

### EXAMPLES

The material for an organic EL device and the organic EL device of the invention will be described in detail referring to the following examples, which should not be construed as limiting the scope of the invention.
The structures of the compounds synthesized or used in the examples are shown below.

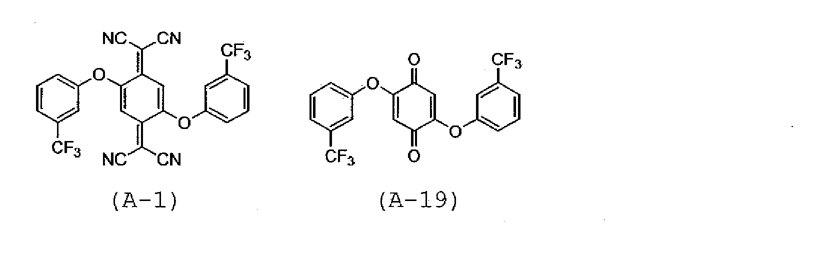

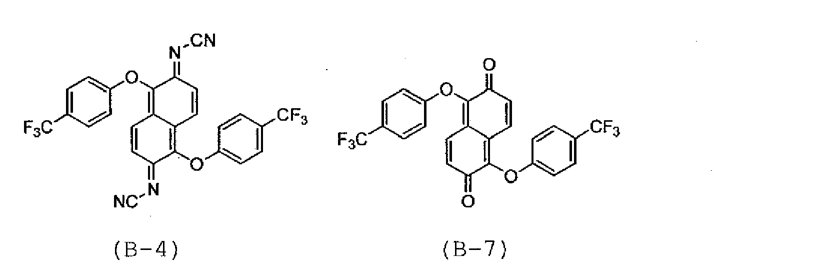

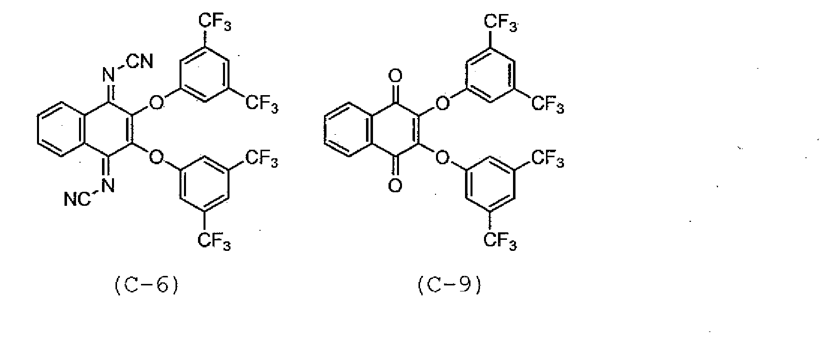

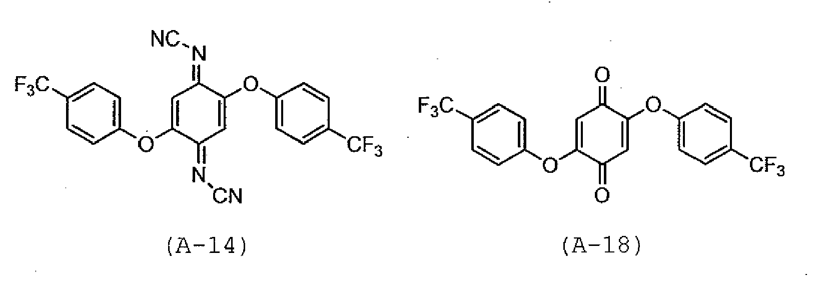

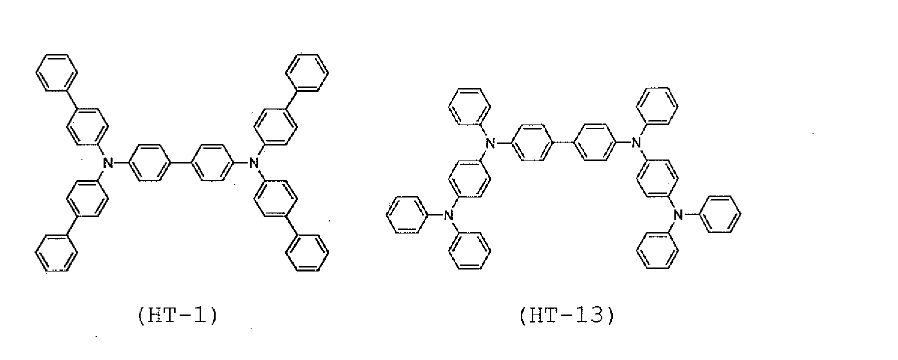

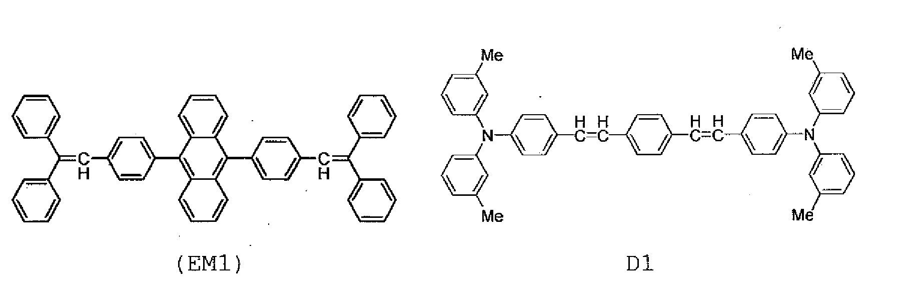

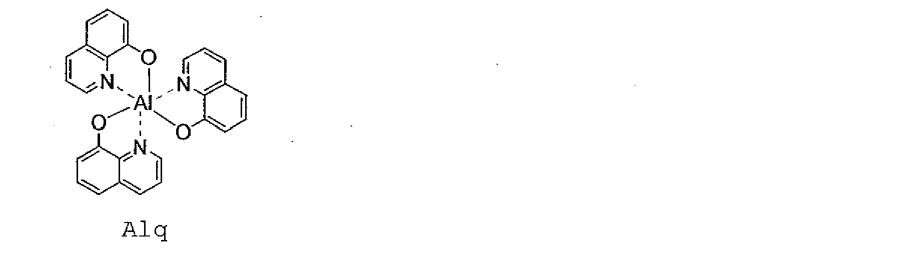

### Example 1

### Synthesis of compound (A-19)

4.5 g of potassium t-butoxide and 10 ml of DMSO were mixed at room temperature under a nitrogen atmosphere, followed by addition of 5.2 g of 3-trifluoromethylphenol. To the resultant mixure, a solution obtained by dissolving 4.3 g of 2,5-dibromobenzoquinone in 15 ml of DMSO was dripped under stirring at room temperature for 8 hours. Thereafter, ethyl acetate and water were added to conduct separation. The organic layer was filtered off by adding sodium sulfuric anhydride, and the solvent was distilled off under a reduced pressure. A residue was purified in a silica gel column, whereby 3.0 g of compound (A-19) was obtained.
As a result of an IR measurement of the compound, absorption of a carbonyl group was observed at 1665 cm⁻¹. Mass spectrometry revealed that the compound had a peak at an M/Z of 428.
The compound was then dissolved in acetonitrile so that the concentration became 0.01 mol/l. A reduction potential was measured by cyclic voltammetry using tetrabutylammonium perchlorate (TBAP) as a supporting electrode and a saturated calomel electrode (SCE) as a reference electrode. The reduction potential obtained by cyclic voltammetry was -0.05 V.

### Example 2

### Synthesis of compound (A-1)

A solution obtained by mixing 1.7 g of the compound (A-19) as obtained above, 0.54 g of malononitrile and methylene chloride was stirred while cooling on ice under a nitrogen atmosphere. Subsequently, 2.4 ml of titanium tetrachloride was dripped, followed by dripping of 3.6 ml of pyridine. After stirring for 5 hours, methylene chloride was distilled off under a reduced pressure, and 5 ml of 1N hydrochloric acid was added. A precipitate was recrystalized from acetonitrile, followed by sublimation and purification, whereby 0.8 g of compound (A-1) was obtained.
As a result of an IR measurement of the compound, absorption of a cyano group was observed at 2222 cm⁻¹. Mass spectrometry revealed that the compound had a peak at an M/Z of 524.
The compound was then dissolved in acetonitrile so that the concentration became 0.01 mol/l. A reduction potential was measured by cyclic voltammetry using tetrabutylammonium perchlorate (TBAP) as a supporting electrode and a saturated calomel electrode (SCE) as a reference electrode. The reduction potential obtained by cyclic voltammetry was 0.33 V.

### Example 3

### Synthesis of compound (B-7)

2.7 g of compound (B-7) was obtained in substantially the same manner as in Example 1, except that 5.0 g of 1,5-dibromo-2,6-naphthoquinone was used instead of 2,5-dibromobenzoquinone and 5.2 g of 4-trifluoromethylphenol was used instead of 3-trifluoromethylphenol.
As a result of an IR measurement of the compound, absorption of a carbonyl group was observed at 1658 cm⁻¹. Mass spectrometry revealed that the compound had a peak at an M/Z of 478.
The reduction potential obtained by cyclic voltammetry was 0.01 V.

### Example 4

### Synthesis of compound (B-4)

Under a nitrogen atmosphere, 40 ml of methylene chloride and 1.7 g of titanium tetrachloride were mixed with 1.1 g of the compound (B-7) as obtained above. To the resultant solution, a solution obtained by mixing 4.1 g of bistrimethylsilyl carbodimide with 10 ml of methylene chloride was dripped while cooling in an ice bath. Subsequently, stirring was continued at room temperature for 8 hours. After the reaction was completed, methylene chloride and water were added to the mixure to extract an organic layer. The methylene chloride solution was concentrated and purified in a silica gel column, followed by sublimation and purification, whereby 0.5 g of compound (B-4) was obtained.
As a result of an IR measurement of the compound, absorption of a cyano group was observed at 2133 cm⁻¹. Mass spectrometry revealed that the compound had a peak at an M/Z of 526.
The reduction potential obtained by cyclic voltammetry was 0.45 V.

### Example 5

### Synthesis of compound (C-9)

3.2 g of compound (C-9) was obtained in substantially the same manner as in Example 1, except that 5.0 g of 2,3-dibromo-1,4-naphthoquinone was used instead of 2,5-dibromobenzoquinone and 7.4 g of 3,5-bis(trifluoromethyl)phenol was used instead of 3-trifluoromethylphenol.
As a result of an IR measurement of the compound, absorption of a carbonyl group was observed at 1655 cm⁻¹. Mass spectrometry revealed that the compound had a peak at an M/Z of 614.
The reduction potential obtained by cyclic voltammetry was -0.05 V.

### Example 6

### Synthesis of compound (C-6)

0.4 g of compound (C-6) was obtained in substantially the same manner as in Example 4, except that the compound (C-9) synthesized in Example 5 was used instead of the compound (B-7).
As a result of an IR measurement of the compound, absorption of a cyano group was observed at 2130 cm⁻¹. Mass spectrometry revealed that the compound had a peak at an M/Z of 662.
The reduction potential obtained by cyclic voltammetry was 0.39 V.

### Example 7

### Synthesis of compound (A-18)

3.2 g of compound (A-18) was obtained in substantially the same manner as in Example 1, except that 5.2 g of 4-trifluoromethylphenol was used instead of 3-trifluoromethylphenol.
As a result of an IR measurement of the compound, absorption of a carbonyl group was observed at 1665 cm⁻¹. Mass spectrometry revealed that the compound had a peak at an M/Z of 428.
The reduction potential obtained by cyclic voltammetry was -0.02 V.

### Example 8

### Synthesis of compound (A-14)

0.6 g of compound (A-14) was obtained in substantially the same manner as in Example 4, except that the compound (A-18) synthesized in Example 7 was used instead of the compound (B-7).
As a result of an IR measurement of the compound, absorption of a cyano group was observed at 2125 cm⁻¹. Mass spectrometry revealed that the compound had a peak at an M/Z of 476.
The reduction potential obtained by cyclic voltammetry was 0.39 V.

### Example 9

A grass substrate of 25 mm by 75 mm by 1.1 mm thick with an ITO transparent electrode (anode) (GEOMATEC CO., LTD.) (thickness of ITO was 130 nm) was subjected to ultrasonic cleaning with isopropyl alcohol for 5 minutes, and cleaned with ultraviolet rays and ozone for 30 minutes.
The cleaned glass substrate having the transparent electrode lines was then secured to a substrate holder of an apparatus for vacuum deposition. First, the compound represented by the formula (A-1) synthesized in Example 2 and a compound represented by the above-mentioned formula (HT-1) were deposited onto the surface of the glass substrate on which the transparent electrode lines were formed so as to cover the transparent electrodes, thereby forming a 60 nm-thick film in which the compound represented by the formula (A-1) and the compound represented by the above-mentioned formula (HT-1) were mixed at a molar ratio of 2:98. The film of the compound mixture served as a hole-injecting layer.

Subsequently, a 20 nm-thick film of a compound represented by the above-mentioned formula (HT-13) was formed on the above-obtained film of the compound mixture. The film served as a hole-transporting layer.
Further, a compound represented by the above-mentioned formula (EM1) with a thickness of 40 nm was deposited thereon to form a film. Simultaneously, the above-mentioned compound (D1) was deposited such that the weight ratio of EM1 and D1 became 40:2. The film served as an emitting layer.
A compound (Alq) represented by the above formula was deposited to form a 10 nm thick film on the above-obtained film. The film serves as an electron-injecting layer. Then, Li as a reductive dopant (Li source: manufactured by SAES Getters Co., Ltd.) and Alq were co-deposited, whereby an Alq:Li film (film thickness: 10 nm) was formed as an electron-injecting layer (cathode). Metal aluminum was deposited on the Alq:Li film to form a metallic cathode, whereby an organic EL emitting device was fabricated.

The organic EL device was evaluated by measuring a driving voltage at a current density of 10 mA/cm² and a half life of luminance at an initial luminance of 1,000 nits, at room temperature, and with a DC constant power supply. The results obtained are shown in Table 1.

### Example 10

An organic EL device was fabricated and evaluated in substantially the same manner as in Example 9, except that the hole-injecting layer was formed using the compound (B-4) synthesized in Example 4 singly instead of the compounds (A-1) and (HT-1). The results are shown in Table 1.

### Example 11

An organic EL device was fabricated and evaluated in substantially the same manner as in Example 9, except that the compound (C-6) synthesized in Example 9 was used instead of the compound (A-1) and the compound (HT-13) was used instead of the compound (HT-1). The results are shown in Table 1.

### Example 12

An organic EL device was fabricated and evaluated in substantially the same manner as in Example 9, except that the compound (A-14) synthesized in Example 8 was used instead of the compound (A-1). The results are shown in Table 1.

### Comparative Example 1

An organic EL device was fabricated and evaluated in the same manner as in Example 9, except that the hole-injecting layer was formed using a compound represented by the formula (HT-1) singly. The results are shown in Table 1.

**Table 1**

| | Constitution material for hole-injection layer | Driving voltage (V) | Half life (hr) |
|---|---|---|---|
| Example 9 | Formula (A-1) Formula (HT-1) | 5.8 | 6,700 |
| Example 10 | Formula (B-4) | 5.5 | 6,200 |
| Example 11 | Formula (C-6) Formula (HT-13) | 5.9 | 6,500 |
| Example 12 | Formula (A-14) Formula (HT-1) | 5.7 | 6,600 |
| Comparative Example 1 | Formula (HT-1) | 6.6 | 5,000 |

### INDUSTRIAL APPLICABILITY

The material for an organic EL device of the invention is suitable as a constitution material of an organic EL device, in particular, a hole-transporting layer or a hole-injecting layer. The material for an organic EL device of the invention can also be used as a charge-transporting material of an electrophotographic photoreceptor. In addition, the material is useful as a material for an organic photoreceptor or as a material for an organic solar battery.
The organic EL device of the invention can be suitably used as a light source such as a planar emitting material and backlight of a display, a display part of a portable phone, PDA, a car navigator, or an instruction panel of an automobile, an illuminator, and the like.

## Claims

1. A material for an organic electroluminescent device comprising a quinone derivative represented by the following formula (1), (2) or (3): 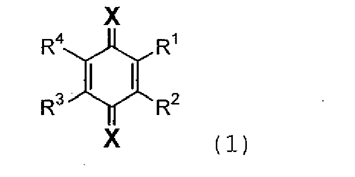 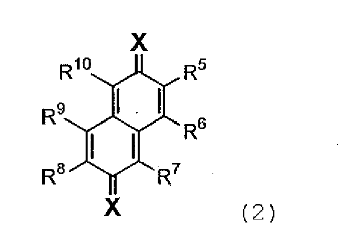 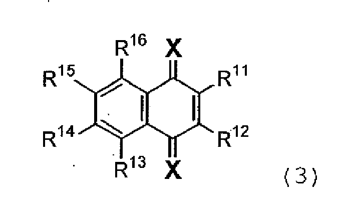 wherein R¹ to R¹⁶ are each a hydrogen atom, a halogen atom, a cyano group, an alkoxy group, a substituted or unsubstituted aryloxy group, an alkyl group, a fluoroalkyl group, an aryl group or a heterocyclic group; provided that at least one of R¹ to R⁴, at least one of R⁵ to R¹⁰ or at least one of R¹¹ to R¹⁶ is an aryloxy group; and X is a substituent represented by any one of the following formulas (a) to (f): 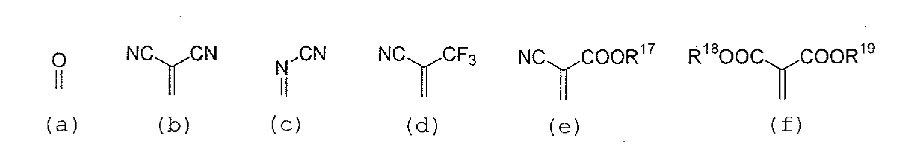 wherein R¹⁷ to R¹⁹ are a hydrogen atom, an alkyl group, or aryl group; and R¹⁸ and R¹⁹ may be bonded together to form a ring.

2. An organic electroluminescent device comprising:
an anode,
a cathode, and
one or a plurality of organic thin layer(s) comprising an emitting layer, interposed between the anode and the cathode;
at least one layer of the organic thin layer(s) containing the material for an organic electroluminescent device of claim 1.

3. The organic electroluminescent device according to claim 2 wherein the organic thin layers are a multilayer body in which a hole-transporting layer, an emitting layer and an electron-transporting layer are stacked in this order from the anode.

4. The organic electroluminescent device according to claim 3 wherein the hole-transporting layer contains the material for an organic electroluminescent device.

5. The organic electroluminescent device according to claim 2 wherein the organic thin layers are a multilayer body in which a hole-injecting layer, a hole-transporting layer, an emitting layer and an electron-transporting layer are stacked in this order from the anode;
the hole-injecting layer containing the material for an organic electroluminescent device.

6. The organic electroluminescent device according to claim 4 or 5 wherein the hole-transporting layer or the hole-injecting layer containing the material for an organic electroluminescent device further contains a phenylenediamine compound represented by the following formula (4): 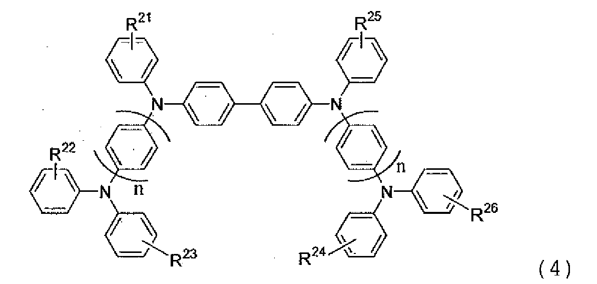 wherein R²¹ to R²⁶ are a hydrogen atom, a halogen atom, a trifluoromethyl group, an alkyl group, an aryl group or a heterocyclic group; R²¹ to R²⁶ may form a naphthalene skeleton, a carbazole skeleton, or a fluorene skeleton with a phenyl group bonded; and n represents 1 or 2.

7. A quinone derivative represented by the following formula (5), (6) or (7): 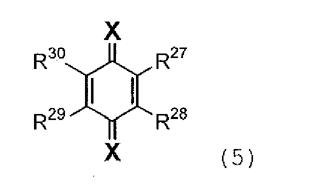 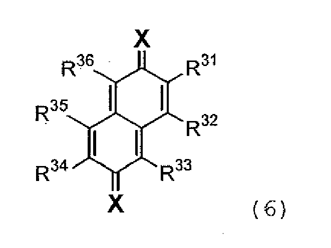 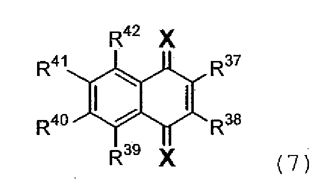 wherein R²⁷ to R⁴² are each a hydrogen atom, a halogen atom, a cyano group, an alkoxy group, a substituted or unsubstituted aryloxy group, an alkyl group, a fluoroalkyl group, an aryl group or a heterocyclic group; provided that at least one of R²⁷ to R³⁰, at least one of R³¹ to R³⁶ or at least one of R³⁷ to R⁴² is a fluorine atom or an aryloxy group having a fluoroalkyl group; and X is a substituent represented by any one of the following formulas (a) to (f): 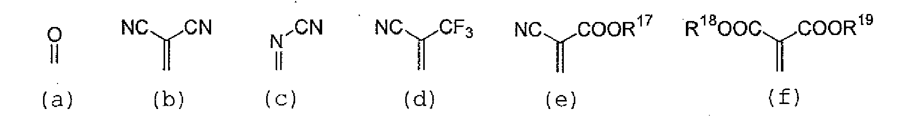 wherein R¹⁷ to R¹⁹ are a hydrogen atom, an alkyl group, or aryl group; and R¹⁸ and R¹⁹ may be bonded together to form a ring.
